(19) Europäisches Patentamt
European Patent Office
Office européen des brevets

(11) Veröffentlichungsnummer: **0 034 323**
**B1**

(12) # EUROPÄISCHE PATENTSCHRIFT

(45) Veröffentlichungstag der Patentschrift: **17.08.83**

(51) Int. Cl.³: **C 07 C 103/52**, C 07 C 101/19, C 07 C 139/00, C 07 C 143/78, C 12 Q 1/00

(21) Anmeldenummer: **81100909.1**

(22) Anmeldetag: **10.02.81**

(54) **Aminosäure- und Peptidester von Leuko-Indoanilinen, Verfahren zu deren Herstellung sowie diese Verbindungen enthaltende Mittel zum Nachweis proteolytischer Enzyme.**

(30) Priorität: **16.02.80 DE 3005845**

(43) Veröffentlichungstag der Anmeldung: **26.08.81 Patentblatt 81/34**

(45) Bekanntmachung des Hinweises auf die Patenterteilung: **17.08.83 Patentblatt 83/33**

(84) Benannte Vertragsstaaten: **AT BE CH DE FR GB IT LI LU NL SE**

(56) Entgegenhaltungen:
**DE-A-2 854 987**
**Chemical Abstracts, Band 79, Nr. 10, 1973, Columbus, Ohio, USA, R. STEINER et al., »Stabilized polyesters«, Seite 50, Spalte 1, Abstract Nr. 54426t**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Berger, Dieter, Dr.rer.nat., Bensheimer-Strasse 45, D-6806 Viernheim (DE)**
Erfinder: **Braun, Franz, Dr.rer.nat., Ringstrasse 14, D-6149 Rimbach 1/Odw. (DE)**
Erfinder: **Frey, Günter, Heinigstrasse 45, D-6700 Ludwigshafen (DE)**
Erfinder: **Knappe, Wolfgang-Reinhold, Dr.rer.nat., Karlsbader Strasse 3, D-6842 Bürstadt 1 (DE)**
Erfinder: **Kuhr, Manfred, Dr.rer.nat., Werthmannweg 23, D-6800 Mannheim-31 (DE)**
Erfinder: **Werner, Wolfgang, Dr.rer.nat., Meissener Weg 39, D-6800 Mannheim-Vogelstang (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 034 323 B1

BUNDESDRUCKEREI BERLIN

Aminosäure- und Peptidester von Leuko-Indoanilinen, Verfahren zu deren Herstellung sowie diese Verbindungen enthaltende Mittel zum Nachweis proteolytischer Enzyme

Der Nachweis von Leukozyten in Körperflüssigkeiten, insbesondere im Urin, nimmt eine hervorragende Stelle in der Diagnostik der Erkrankungen der Niere und des Urogenitaltraktes ein.

Bisher wird dieser Nachweis geführt durch mühsames Auszählen der Leukozyten im nicht zentrifugierten Harn oder im Harnsediment.

Beiden Methoden ist naturgemäß gemeinsam, daß nur intakte Leukozyten erfaßt werden. Andererseits ist bekannt, daß die Geschwindigkeit der Leukozyten-Lyse je nach Harnmilieu enormen Schwankungen unterworfen ist; so ist z. B. in stark alkalischen Harnen mit einer Leukozyten-Halbwertszeit von nur 60 Minuten zu rechnen. Zu niedrige Leukozytenzahlen bzw. bei längeren Harnstandzeiten sogar falschnegative Befunde sind die Folge.

Vom Lyse-Fehler abgesehen, liefert die quantitative mikroskopische Bestimmung der Leukozyten im nicht zentrifugierten, homogenisierten Harn in der Zählkammer recht zuverlässige Werte. In der Praxis wird diese Methode jedoch nur selten angewandt, da sie mühevoll, ermüdend und zeitraubend ist und den Einsatz geschulten Personals bedingt.

Die überwiegende Mehrzahl der Leukozytenbestimmungen im Harn werden in der medizinischen Praxis nach der sogenannten Gesichtsfeldmethode im Harnsediment durchgeführt. Hierzu muß zunächst das Untersuchungsgut (Sediment) durch Zentrifugieren gewonnen werden. Dabei werden jedoch auch andere Bestandteile des Harnes angereichert, die — wie z. B. Salze und Epithelzellen — die mikroskopische Auszählung der Leukozyten beträchtlich erschweren können. Schwankender Sedimentgehalt, Inhomogenitäten des Sediments sowie womöglich unterschiedliche mikroskopische Vergrößerungen oder unterschiedliche optische Ausstattung der Mikroskope führen dazu, daß die hier übliche Angabe über die Anzahl der Leukozyten pro mikroskopischem Gesichtsfeld mit Fehlern von mehreren hundert Prozent behaftet sein kann.

Aufgabe der vorliegenden Erfindung war es daher, ein diagnostisches Mittel bereitzustellen, mit dem die Leukozyten in Körperflüssigkeiten auf einfache und leicht zu handhabende Weise sowie möglichst schnell und vollständig nachgewiesen werden können.

Als Nachweisprinzip für einen solchen Leukozytentest bietet sich eine enzymatische Reaktion an, da die Leukozyten ein breitgefächertes Enzymspektrum besitzen.

In dem US-Patent 3 087 794 ist bereits ein Leukozytennachweis beschrieben und beansprucht, der über die in den granulozytären Leukozyten vorhandene peroxidatische Aktivität geführt wird. Ein saugfähiger Träger, der mit Wasserstoffperoxid und einem organischen Indikator, beispielsweise o-Tolidin, imprägniert ist, zeigt die Anwesenheit von Leukozyten durch die Bildung eines farbigen Oxidationsproduktes an. Ein solcher Test besitzt jedoch entscheidende Nachteile: Zum einen besitzen peroxidatische Reaktionen unter Verwendung von o-Tolidin ganz allgemein gegenüber reduzierenden Substanzen im Harn, wie z. B. gegenüber Ascorbinsäure, eine erhebliche Störanfälligkeit. Darüber hinaus finden sich in mehreren Literaturstellen (siehe z. B. L. Mettler, Med. Welt, 23, 399 [1972]) Hinweise auf die Instabilität der Leukozytenperoxidase im Harnmilieu, die zu falschnegativen Befunden Anlaß gibt. Noch gravierender ist die zu erwartende mangelhafte Selektivität dieses Testes gegenüber Erythrozyten.

Seit einigen Jahren haben in der histo- und zytochemischen Enzymologie Nachweismethoden ihren festen Platz, die auf der esterolytischen Aktivität der in den zu bestimmenden Systemen vorhandener Enzyme beruhen (vgl. zum Beispiel A. G. E. Pearse, Histochemistry, Theoretical and Applied, 3. Ed., Churchill Livingstone, Edinburgh—London—New York, 1968). Im Prinzip werden dabei farblose oder schwach gefärbte Ester eingesetzt, die durch die enzymatische Spaltung zumeist in eine farblose Säure- und eine ebenfalls farblose Alkohol-(Phenol-)Komponente zerfallen. Letztere wird dann in einer der enzymatischen Verseifung folgenden Reaktion zu farbigen Produkten umgesetzt (z. B. Kupplung mit Diazoniumsalzen oder oxidative Reaktionen).

So beschreiben beispielsweise F. Schmalzl und H. Braunsteiner in Klin. Wschr., 46, 642 (1968), einen spezifischen zytochemischen Leukozytenesterase-Nachweis mit Naphthol-AS-D-chloracetat als Substrat und einem Diazoniumsalz zur Bildung der farbigen Azo-Verbindung.

Für ein Mittel zum schnellen und einfachen Nachweis von Leukozyten, beispielsweise im Harn, erweisen sich Zwei-Komponenten-Systeme dieser Art als nicht geeignet, da bekanntlich viele im Harn vorkommende Verbindungen, wie Urobilinogen, Stercobilinogen, Bilirubin u. a., mit Diazoniumsalzen reagieren. Darüber hinaus ist dieser Nachweis viel zu unempfindlich. Beispielsweise zeigen Proben mit 5000 Leukozyten/µl keine Reaktion.

Überraschenderweise wurde nun gefunden, daß man stabile und schnell anzeigende Mittel, mit denen Leukozyten gut in Körperflüssigkeiten nachzuweisen sind, erhält, wenn als Substrate zum Nachweis der in den neutrophilen Leukozyten-Granulozyten vorkommende Esterasen (Proteasen), Aminosäure- oder Peptidester von Leuko-Indoanilinen verwendet werden.

Gegenstand der vorliegenden Erfindung sind daher neue Aminosäure- und Peptidester von Leuko-Indoanilinen der allgemeinen Formel I

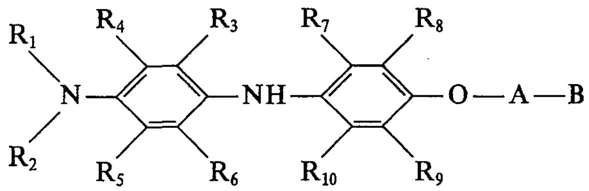

(I)

in der

$R_1$, $R_2$, die gleich oder verschieden sein können, jeweils Wasserstoff, eine niedere Alkyl- oder eine Hydroxy-Niederalkyl-Gruppe oder gemeinsam eine Alkylen- oder Alkylen-oxy-alkylen-Kette,

$R_3$, $R_4$, $R_5$, $R_6$, die gleich oder verschieden sein können, jeweils Wasserstoff oder Halogen, eine niedere, gegebenenfalls vollständig durch Halogen substituierte Alkyl-, eine niedere Alkoxy-, eine Aralkoxy-, eine Hydroxy- oder eine Nitro-Gruppe,

$R_7$, $R_8$, $R_9$, $R_{10}$, die gleich oder verschieden sein können, jeweils Wasserstoff oder Halogen; eine niedere, gegebenenfalls vollständig durch Halogen substituierte Alkyl-, eine niedere Alkoxy-, eine Aralkoxy-, eine eine niedere Acylamino-, eine Acylalkenyl-, eine Hydroxy-, eine niedere Alkylmercapto-, eine niedere Alkylsulfonyl-, eine Carboxy-, eine gegebenenfalls durch eine niedere Alkoxy-, Aralkoxy-, Amino- oder Niederalkyl-Amino-Gruppe substituierte Carbonylgruppe oder jeweils zwei benachbarte Substituenten eine gesättigte oder ungesättigte Kohlenwasserstoffkette, wobei ein Kettenglied durch ein Stickstoffatom ersetzt sein kann,

A einen Aminosäure- oder einen Peptidrest,

B eine in der Peptidchemie übliche oder davon abgeleitete Stickstoffschutzgruppe bedeuten,

sowie Verfahren zur Herstellung dieser Verbindungen und deren Verwendung zur Herstellung von Mitteln zum Nachweis proteolytischer Enzyme.

Die Herstellung der neuen Aminosäure- und Peptidester von Leuko-Indoanilinen der allgemeinen Formel I kann nach an sich aus der Peptidchemie her bekannten Methoden erfolgen.

Vorzugsweise werden in an sich bekannter Weise die entsprechenden Leuko-Indoanilin-Verbindungen der allgemeinen Formel II

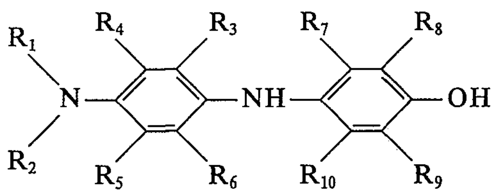

(II)

in der

$R_1$, $R_2$, $R_3$, $R_4$, $R_5$, $R_6$, $R_7$, $R_8$, $R_9$ und $R_{10}$ die oben angegebene Bedeutung haben,

mit Aminosäuren bzw. Peptiden der allgemeinen Formel III

HO—A—B (III)

in der

A und B die oben angegebene Bedeutung haben,

beziehungsweise mit geeigneten reaktiven Derivaten davon, umgesetzt.

Als reaktive Derivate werden z. B. die Säurechloride oder Aktiv-Ester eingesetzt.

Die Indoanilin-Verbindungen bzw. die daraus durch übliche Reduktionsmittel (wie z. B. Natriumsulfit oder Natriumdithionit) hergestellten Leuko-Indoanilin-Substanzen der allgemeinen Formel II sind bekannte Verbindungen, vgl. zum Beispiel P. W. Vittum, G. H. Brown, J. Amer. Chem. Soc., 68, 2235 (1946), und J. Amer. Chem. Soc., 69, 152 (1947), und S. Hünig, P. Richters, Liebigs Ann., 612, 282 (1957), oder können in Analogie zu bekannten Verbindungen hergestellt werden. Dies gilt auch für die Aminosäuren und Peptide der allgemeinen Formel III, vgl. zum Beispiel Houben—Weyl, Methoden der organischen Chemie, Bd. 15/1.

Gegenstand der vorliegenden Erfindung ist ferner ein Mittel zum Nachweis proteolytischer Enzyme, insbesondere zum Nachweis der in den Leukozyten vorhandenen Proteasen, bestehend aus einem saugfähigen Träger, einer Filmschicht, einem Lyophilisat, einer Lösung oder einer Reagenztablette, enthaltend ein oder mehrere Chromogene, eine geeignete Puffersubstanz, sowie gegebenenfalls üblicherweise verwendete Hilfsstoffe, dadurch gekennzeichnet, daß als Chromogene Aminosäure- und/oder Peptidester von Leuko-Indoanilinen der oben angegebenen allgemeinen Formel I eingesetzt werden.

Das Testprinzip des erfindungsgemäßen Mittels beruht auf einer Spaltung der als Chromogene beanspruchten farblosen Aminosäure- und Peptidester von Leuko-Indoanilinen der allgemeinen Formel I durch die Esterasen (Proteasen) zu den praktisch farblosen Leuko-Indoanilin-Verbindungen der allgemeinen Formel II, die in einer Folgereaktion mittels Luftsauerstoff oder einem Oxidationsmittel zu den tiefblaugefärbten Indoanilinen der allgemeinen Formel IIa umgesetzt werden.

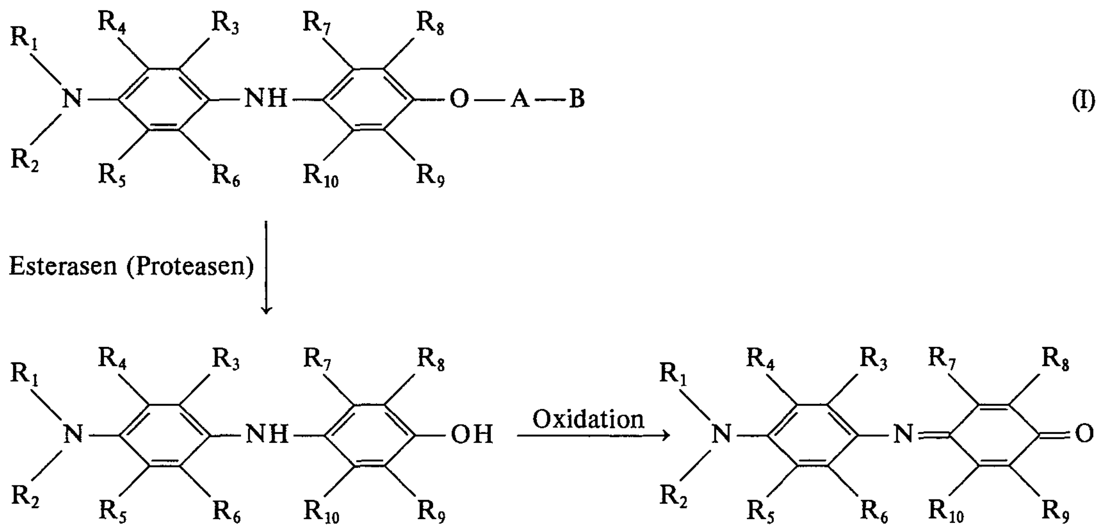


(I)

(II) (IIa)

Die erfindungsgemäßen Substrate sowie die damit hergestellten erfindungsgemäßen Mittel eignen sich ausgezeichnet zum allgemeinen Nachweis proteolytischer Enzyme, insbesondere zum Nachweis der in den Leukozyten vorhandenen Proteasen. Es können damit aber auch andere proteolytische Enzyme, wie beispielsweise Elastase, Chymotrypsin oder Trypsin in rein wäßrigen Lösungen oder auch in Körperflüssigkeiten, wie z. B. Plasma, Serum, Liquor, Pankreassekret oder wäßrigen Stuhlextrakten, nachgewiesen werden.

Die in den Definitionen von $R_1$, $R_2$, $R_3$, $R_4$, $R_5$, $R_6$, $R_7$, $R_8$, $R_9$, $R_{10}$ vorkommenden niederen Alkylreste enthalten 1 bis 6, vorzugsweise 1 bis 4 Kohlenstoffatome, wobei die Methyl-, Äthyl-, n-Butyl- sowie die Methoxy-, Methylmercapto- und Methylsulfonylgruppe ganz besonders bevorzugt sind.

Unter einer »Alkylen-« und »Alkylen-oxy-alkylen-Kette« in der Definition von $R_1$ und $R_2$ sowie unter einer »gesättigten oder ungesättigten Kohlenwasserstoffkette« in der Definition von $R_7$, $R_8$, $R_9$ und $R_{10}$ sind Kohlenwasserstoffreste mit 1 bis 6, vorzugsweise 2 bis 5 Kohlenstoffatomen zu verstehen, die Sauerstoff- bzw. Stickstoffatome als Kettenglieder enthalten können. Besonders bevorzugt sind der Butylen-, der Pentylen-, der Äthylen-oxy-äthylen-, der Butadienylen- und der Aza-Butadienylen-Rest.

»Halogen« in der Definition von $R_3$, $R_4$, $R_5$, $R_6$, $R_7$, $R_8$, $R_9$ und $R_{10}$ bedeutet Fluor, Chlor, Brom oder Jod, vorzugsweise Fluor, Chlor oder Brom, wobei Brom bzw. Fluor ganz besonders bevorzugt sind.

Als »Aralkoxy-Gruppe« in der Definition von $R_3$, $R_4$, $R_5$, $R_6$, $R_7$, $R_8$, $R_9$ und $R_{10}$ kommen durch niedere Alkoxygruppen substituierte Phenyl- und Naphthylreste in Frage, wobei der Alkylrest 1 bis 5, vorzugsweise 1 bis 3 Kohlenstoffatome enthält. Besonders bevorzugt ist der Benzoylrest.

Unter einer »niederen Acylamino-Gruppe« in der Definition von $R_7$, $R_8$, $R_9$ und $R_{10}$ sind die Amidgruppierungen der niederen aliphatischen Carbonsäuren mit 1 bis 5, vorzugsweise 1 bis 3 Kohlenstoffatomen, zu verstehen. Besonders bevorzugt ist der Acetylamino-Rest.

Die »Acylalkenyl-Gruppe« in der Definition von $R_7$, $R_8$, $R_9$, $R_{10}$ bedeutet einen durch Reste aromatischer Carbonsäuren, wie z. B. der Benzoe- oder der Naphthoesäuren, substituierte Alkenyl-Gruppe, wobei die Alkenyl-Gruppe 2 bis 5, vorzugsweise 2 bis 3 Kohlenstoffatome enthält. Besonders bevorzugt ist der Benzoyl-vinyl-Rest.

Als »Aminosäurerest« in der Definition von A kommen vorzugsweise die Reste der natürlichen $\alpha$-Aminosäuren in ihrer L- oder D-Form oder auch in ihrer racemischen Form in Frage. Besonders bevorzugt sind die Reste von Glycin, Alanin, Valin, Serin, Leucin, Methionin, Phenylalanin und Tyrosin. Eine gegebenenfalls vorhandene Hydroxygruppe kann in an sich bekannter Weise, vorzugsweise durch einen Acetyl- oder Benzylrest, geschützt werden.

Unter einem »Peptidrest« in der Definition von A sind z. B. Di-, Tri-, Tetra- und Pentapeptide, vorzugsweise Di- und Tripeptide, zu verstehen, wobei als Aminosäure-Komponenten vorzugsweise die obenerwähnten Aminosäuren Verwendung finden.

Als »in der Peptidchemie übliche Stickstoffschutzgruppe« in der Definition von B sind z. B. Acyl-, Oxycarbonyl- und Sulfonyl-Gruppen zu verstehen.

Die erfindungsgemäß als Chromogene verwendeten Aminosäure- und Peptidester von Leuko-Indoanilinen der allgemeinen Formel I werden in Konzentrationen von $10^{-4}$ bis 1 Mol/l,

vorzugsweise $10^{-3}$ bis $10^{-1}$ Mol/l Imprägnierlösung, Beschichtungsmasse oder zu untersuchender Flüssigkeit eingesetzt.

Ein weiterer Bestandteil des diagnostischen Mittels zum Nachweis proteolytischer Enzyme und insbesondere der Leukozyten-Proteasen ist ein geeignetes Puffersystem. Hierzu kommen z. B. Phosphat-, Borat-, Barbiturat-, Tris-(hydroxymethyl)-aminomethan- (= Tris), 2-Amino-2-methyl-propandiol-1,3- (= Amediol)- oder Aminosäure-Puffer in Frage, wobei pH-Wert und Kapazität so gewählt werden müssen, daß sich in der Meßlösung bzw. auf dem Teststreifen ein pH-Wert von 6–10, vorzugsweise von 7–9, einstellt.

Weiterhin kann man bei der Herstellung des erfindungsgemäßen Mittels zum Nachweis proteolytischer Enzyme, insbesondere der Leukozyten-Proteasen in Körperflüssigkeiten zusätzlich Oxidationsmittel verwenden, um die bei der enzymatischen Reaktion primär entstandenen Leuko-Indoanilin-Verbindungen der allgemeinen Formel II zu den tiefblaugefärbten Indoanilinen der allgemeinen Formel IIa umzusetzen.

Diese Oxidationsmittel, wie z. B. Kalium-hexacyanoferrat-III, Kaliumperoxo-disulfat, Kalium-metaperjodat, Natrium-perborat oder Kaliumchromat werden in Konzentrationen von $10^{-4}$ bis $10^{-1}$ Mol/l, vorzugsweise $10^{-3}$ bis $10^{-2}$ Mol/l Imprägnierlösung, Beschichtungsmasse oder zu untersuchender Flüssigkeit eingesetzt.

Ein weiterer Bestandteil des erfindungsgemäßen Mittels für den Nachweis proteolytischer Enzyme kann ein Netzmittel sein, da hierdurch eine homogenere Farbverteilung und z. T. brillantere Farben erzielt werden können. Es können kationen-, aber auch anionenaktive, sowie amphotere und nichtionogene Netzmittel in Konzentrationen von 0,05–2% (w/v), vorzugsweise 0,1–1% (w/v), eingesetzt werden.

Des weiteren können bei der Herstellung des erfindungsgemäßen Mittels zum Nachweis proteolytischer Enzyme üblicherweise verwendete Antioxidantien (vgl. zum Beispiel Ullmanns Enzyklopädie der technischen Chemie, Bd. 8), wie z. B. solche des Phenoltyps zugesetzt werden, da hierdurch die Stabilität der erfindungsgemäß verwendeten Aminosäure- und Peptidester gegenüber oxidativen Zersetzungsprozessen erheblich verbessert wird. Die Antioxidantien finden dabei sowohl als Monokomponenten, wie auch als synergistische Systeme (z. B. Hydrochinon plus Hydrochinonmonoalkyläther) Anwendung. Eingesetzt werden die Antioxidantien in Konzentrationen von $10^{-4}$ bis $10^{-2}$ Mol/l, wobei die Konzentration so zu wählen ist, daß der der enzymatischen Spaltung der Aminosäureester zu den Leuko-Indoanilin-Verbindungen folgende oxidative Sekundärschritt zu den gefärbten Indoanilinen nicht gestört wird.

Als weiterer Bestandteil des erfindungsgemäßen Mittels kann als Stabilisator ein Phosphor- bzw. Phosphonsäureamid der allgemeinen Formel IV dienen

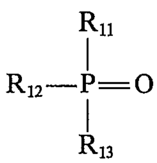

(IV)

in der

$R_{11}$ eine Dialkylamino-, eine Alkoxy-, eine Aryloxy-, eine Alkyl- oder eine Arylgruppe oder einen N-Morpholinrest und

$R_{12}$ und $R_{13}$ eine Dialkylaminogruppe oder einen N-Morpholinrest bedeuten.

Als »Alkoxy- bzw. Alkylgruppe« in der Definition von $R_{11}$ kommen Kohlenwasserstoffreste bis 10 Kohlenstoffatomen in Frage.

Unter »Aryl- bzw. Aryloxygruppe« in der Definition von $R_{11}$ sind gegebenenfalls durch Halogen, niedere Alkyl- oder Alkoxygruppen substituierte Phenyl- oder Naphthylreste zu verstehen.

Mit Hilfe der Verbindungen der allgemeinen Formel IV gelingt eine erstaunliche Stabilisierung der Rezepturen.

Die Phosphor- und Phosphonsäureamide der allgemeinen Formel IV sind bekannte Verbindungen, sie finden z. B. in der deutschen Auslegeschrift 2 235 127 Anwendung als Stabilisatoren von Teststreifenrezepturen, die auf der Basis eines Peroxidasenachweises arbeiten.

Die Stabilisatoren des Phosphor- und Phosphonsäureamid-Typs der allgemeinen Formel IV werden der wäßrigen Imprägnierlösung in Konzentrationen von 1–20% (w/v), vorzugsweise 5–15% (w/v), zugesetzt.

Überraschenderweise wurde gefunden, daß die Reaktionszeiten des erfindungsgemäßen diagnostischen Mittels zum Nachweis proteolytischer Enzyme, insbesondere proteolytischer Leukozytenenzyme, erheblich verkürzt werden können, wenn zusätzlich zu den bisher angeführten Chromogenen und Hilfsstoffen ein oder mehrere Aktivatoren eingesetzt werden. Geeignete Aktivatoren sind beispielsweise

a) Alkohole der allgemeinen Formel V,

$R_{14}-K-OH$ (V)

in der

$R_{14}$ Wasserstoff, eine Hydroxy- oder eine niedere Alkoxygruppe und
K einen Kohlenwasserstoffrest bedeuten,

b) Metallkomplexe der allgemeinen Formel VI,

$D_m[M(CN)_n(NO)_p]$ (VI)

in der

D ein Alkalimetallion,
M ein Schwermetallion,
m eine ganze Zahl von 2 bis 5,
n eine ganze Zahl von 4 bis 8 und
p 0 oder 1 bedeuten.

Die »niedere Alkoxygruppe« in der Definition von $R_{14}$ enthält 1 bis 5, vorzugsweise 3 bis 4 Kohlenstoffatome. Besonders bevorzugt ist die Butyloxy-Gruppe.

Der Kohlenwasserstoffrest K in der Definition von $R_{14}$ kann geradkettig oder verzweigt, gesättigt oder ungesättigt, cyclisch oder acyclisch sein und enthält 1 bis 30, vorzugsweise 2 bis 22 Kohlenstoffatome bei den acyclischen Verbindungen und 3 bis 20, vorzugsweise 6 bis 17 Kohlenstoffatomen bei den cyclischen Verbindungen.

In den Aktivatoren der allgemeinen Formel VI kommen als Alkalimetallionen D vorzugsweise Natrium- und Kalium-Ionen und als Schwermetallionen M vorzugsweise Ionen der Metalle Eisen, Nickel, Chrom, Mangan, Kobalt, Molybdän und Vanadium in Frage.

Als im Sinne der vorliegenden Erfindung einsetzbare Aktivatoren seien beispielhaft erwähnt:

1. Hexanol-(1)
2. Heptanol-(1)
3. Octanol-(1)
4. Nonanol-(1)
5. Decanol-(1)
6. Dodecanol-(1)
7. Tetradecanol-(1)
8. Pentadecanol-(1)
9. Hexadecanol-(1)
10. Heptadecanol-(1)
11. Octadecanol-(1)
12. Nonadecanol-(1)
13. Eicosanol-(1)
14. Docosanol-(1)
15. Cyclohexanol
16. Cyclohexen-(1)-ol-(1)
17. Cycloheptanol
18. Cyclooctanol
19. Cyclononanol
20. Cyclodecanol
21. Cyclododecanol
22. Cycloheptadecanol
23. Cycloheptadecen-(9)-ol-(1)
24. Citronellol
25. Geraniol
26. Nerol
27. Linalool
28. Farnesol
29. Nerolidol
30. cis-Octadecen-(9)-ol-(1)
31. Phytol
32. Pentandiol-(1,5)
33. Hexandiol-(1,6)

34. Heptandiol-(1,7)
35. Octandiol-(1,8)
36. Nonandiol-(1,9)
37. Decandiol-(1,10)
38. Dodecandiol-(1,12)
39. 2-Butyloxyethanol
40. 2-(2-Butyloxyethyloxy)ethanol
41. Tri-kalium-hexacyano-ferrat-III
42. Tetra-kalium-hexacyano-ferrat-II
43. Di-kalium-tetracyano-nickelat-II
44. Tri-natrium-octacyano-molybdat-V
45. Di-natrium-pentacyano-nitrosyl-ferrat-II
46. Tri-kalium-pentacyano-nitrosyl-manganat-I
47. Tri-kalium-pentacyano-nitrosyl-chromat-I
48. Tri-kalium-pentacyano-nitrosyl-kobaltat-I
49. Penta-kalium-pentacyano-nitrosyl-vanadat-I

Sämtliche erfindungsgemäß als Aktivatoren verwendeten Verbindungen sind bekannt.

Die Aktivatoren der allgemeinen Formel V werden in Konzentrationen von 0,5 – 10%, vorzugsweise 1 – 5% (w/v), Aktivatoren der allgemeinen Formel VI in Konzentrationen von $10^{-4}$ bis 1 Mol/l, vorzugsweise $10^{-3}$ bis $10^{-1}$ Mol/l Imprägnierlösung eingesetzt.

Zur Herstellung des erfindungsgemäßen Mittels werden z. B. saugfähige Träger, vorzugsweise Filterpapier, Cellulose oder Kunstfaservliese, mit Lösungen der erforderlichen, üblicherweise zur Herstellung von Teststreifen verwendeten Reagenzien (Substrat, Puffer, gegebenenfalls Netzmittel, Oxidationsmittel usw.) in leichtflüchtigen Lösungsmitteln, wie z. B. Wasser, Methanol, Ethanol oder Aceton, imprägniert. Dies geschieht zweckmäßig in zwei getrennten Schritten: Zunächst wird mit einer wäßrigen Lösung imprägniert, die den Puffer und andere wasserlösliche Zusatzstoffe enthält. Danach wird mit einer Lösung der Protease-Substrate der allgemeinen Formel I imprägniert. In speziellen Fällen kann auch die umgekehrte Imprägnierfolge angewandt werden.

Die fertigen Testpapiere können als solche verwendet werden oder in an sich bekannter Weise an Griffen angeklebt oder vorzugsweise zwischen Kunststoffen und feinmaschigen Netzwerken gemäß DE-PS 2 118 455 eingesiegelt werden.

Zur Herstellung filmbeschichteter Teststreifen werden sämtliche Reagenzien in die Lösung oder Dispersion einer filmbildenden Substanz, wie z. B. Polyvinylester oder Polyamid, eingetragen und homogen vermischt. Das Gemisch wird in dünner Schicht auf einen Kunststoffträger gestrichen und getrocknet. Die erfindungsgemäßen filmbeschichteten Teststreifen werden nach dem Trocknen geschnitten und können als solche verwendet werden oder in an sich bekannter Weise an Griffen angeklebt werden oder z. B. zwischen Kunststoffen und feinmaschigen Netzwerken gemäß DE-PS 2 118 455 eingesiegelt werden.

Das erfindungsgemäße diagnostische Mittel zum Nachweis proteolytischer Enzyme, insbesondere der Leukozyten-Proteasen, in Form von Pulvermischungen oder Reagenztabletten läßt sich herstellen, indem die oben angeführten Bestandteile des Testes mit üblichen galenischen Zusatzstoffen versetzt und granuliert werden. Zusatzstoffe dieser Art sind z. B. Kohlenhydrate, wie z. B. Mono-, Oligo- oder Polysaccharide, oder Zuckeralkohole, wie z. B. Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 50 – 200 mg, vorzugsweise 50 – 80 mg, auf.

Zur Herstellung von Lyophilisaten im Gesamtgewicht von jeweils etwa 5 – 20 mg, vorzugsweise etwa 10 mg, wird eine Lösung gefriergetrocknet, die neben sämtlichen für den Test benötigten Reagenzien übliche Gerüstbildner, wie z. B. Polyvinylpyrrolidon, und evtl. weitere Füllstoffe, wie z. B. Mannit, Sorbit oder Xylit, enthält.

Das erfindungsgemäße diagnostische Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z. B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden.

Die so hergestellten diagnostischen Mittel ermöglichen es, nach Eintauchen in die zu untersuchende oder nach Zugabe zur betreffenden Körperflüssigkeit die Anwesenheit proteolytischer Enzyme, insbesondere der Leukozyten-Proteasen, rasch und einfach über eine Farbbildung nachzuweisen, die visuell oder photometrisch, z. B. remissionsphotometrisch oder in der Küvette, beurteilt werden kann. Da die Aktivität der Leukozyten-Proteasen pro Zelle als eine im wesentlichen konstante Größe angesehen werden kann, läßt sich aus der Intensität der Farbbildung die Leukozytenkonzentration der untersuchten Körperflüssigkeit ermitteln. Dabei werden mit dem erfindungsgemäßen diagnostischen Mittel sowohl intakte als auch lysierte Leukozyten erfaßt, da die Aktivität der Leukozyten-Proteasen

auch nach der Lyse der Leukozyten voll erhalten bleibt. Ein Lysefehler tritt folglich nicht auf.

## Beispiel 1

4-[N-(Toluol-4″-sulfonyl)-L-alanyloxy]-4′-dimethylamino-diphenylamin

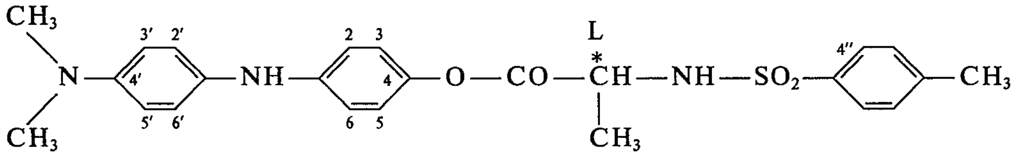

Lösung 1:

Zur Herstellung des Säurechlorids nach der Einstufen-Methode werden 4,739 g (0,0195 Mol) N-(Toluol-4-sulfonyl)-L-alanin in 35 ml abs. Dimethylformamid (DMF) gelöst und auf −20° C abgekühlt. Dann werden unter Rühren und Kühlen 1,645 ml (0,0195 Mol) Thionylchlorid zupipettiert und das Reaktionsgemisch 30 Minuten im Kältebad bei −20° C belassen.

Lösung 2:

In einer Schutzgasatmosphäre (Stickstoff) wird eine Lösung von 2,964 g (0,013 Mol) 4-Hydroxy-4′-dimethylamino-diphenylamin in 30 ml abs. DMF versetzt mit 1,79 ml (0,013 Mol) Triäthylamin und 4,2 ml (0,052 Mol) Pyridin. Das Gemisch wird auf −20° C abgekühlt.

Umsetzung:

Man gießt Lösung 1 zu Lösung 2 und rührt unter Sauerstoff- und Wasserausschluß ca. 5 Stunden bei −20° C, hernach läßt man das Gemisch allmählich über Nacht auf Kühlschranktemperatur kommen.

Zur Aufarbeitung wird das Reaktionsgemisch in Vakuum bei maximal 40−50° C Badtemperatur eingeengt. Der Rückstand wird in etwa 100 ml Essigester aufgenommen und nacheinander dreimal mit 50 ml 5%iger Natriumbicarbonatlösung und einmal mit 50 ml 5%iger Zitronensäure gewaschen. Nach Trocknen der Essigesterphase über Natriumsulfat wird diese im Vakuum eingedampft. Das so erhaltene ölige Rohprodukt wird säulenchromatographisch an Kieselgel mit einem Toluol-Essigester-Gemisch 1 : 1 gereinigt. Nach dem Abdestillieren des Lösungsmittels der gesammelten Fraktionen im Vakuum erhält man durch Anrühren des Rückstandes in Ether das kristalline Produkt. Nach dem Absaugen wird der Rückstand in sehr wenig Methylenchlorid gelöst und mit Ether gefällt. Man erhält so

0,8 g (13,6%) 4-[N-(Toluol-4″-sulfonyl)-L-alanyloxy]-4′-dimethylamino-diphenylamin, farblose Kristalle, Schmp. 149° C; $\alpha_D^{20}$: −61,1°, c = 1% (Aceton).

In analoger Weise erhält man durch Umsetzung der entsprechend substituierten Diphenylamin-Verbindungen (Leuko-Indoaniline) mit den jeweiligen N-geschützten Aminosäuren die folgenden Substrate:

1.1 2,6-Dimethyl-4-[N-(Toluol-4″-sulfonyl)-L-alanyloxy]-4′-dimethylamino-diphenylamin, farblose Kristalle, Schmp. 124−126° C; $\alpha_D^{20}$: −40,4°, c = 1% (Aceton).

1.2 3,5-Dimethyl-4-[N-(Toluol-4″-sulfonyl)-L-alanyloxy]-4′-dimethylamino-diphenylamin, schwachgraublaue Kristalle, Schmp. 186−189° C.
DC: Fertigplatte Kieselgel
(Laufmittel: Toluol-Essigester 1 : 2.
Detektion: UV, $NH_3$ [Gas] und Kaliumhexacyanoferrat-III,
$R_F$-Wert: 0,53).

1.3 4-[N-(Toluol-4″-sulfonyl)-L-alanyloxy]-4′-diethylamino-diphenylamin, farblose Kristalle, Schmp. 117−120° C; $\alpha_D^{20}$: −69,3°, c = 1% (Aceton).

Beispiel 2

4-[N-(Toluol-4″-sulfonyl)-L-alanyloxy]-4′-di-(n-butyl)-aminodiphenylamin

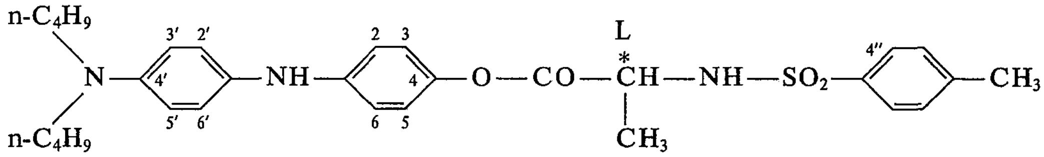

Zur Umsetzung nach dem Aktivester-Verfahren wird zunächst ein Gemisch von 2,9 g (0,012 Mol) N-(Toluol-4-sulfonyl)-L-alanin, 3,24 g (0,018 Mol) N-Hydroxy-benzotriazol und 2,59 g (0,012 Mol) Dicyclohexylcarbodiimid in 60 ml Essigester zunächst 2 Stunden bei 0°C, dann 2 Stunden bei Raumtemperatur gerührt.

Zu dem so gebildeten Aktivester fügt man dann unter Sauerstoff- und Wasserausschluß 2,49 g (0,008 Mol) 4-Hydroxy-4′-di-(n-butyl)-aminodiphenylamin und 1,1 ml (0,008 Mol) Triethylamin hinzu. Das Gemisch wird ca. 15 Stunden bei Raumtemperatur gerührt. Nach Absaugen des gebildeten N,N′-Dicyclohexylharnstoffes wird das Filtrat wie in Beispiel 1 beschrieben aufgearbeitet. Das ölige Rohprodukt wird dann säulenchromatographisch an Kieselgel mit einem Toluol-Essigester-Gemisch 4 : 1 gereinigt. Nach dem Einengen des Lösungsmittels der gesammelten Fraktionen wird der verbleibende ölige Rückstand mit einem Ether-Ligroin-Gemisch angerieben. Das so erhaltene Produkt wird weiter gereinigt durch Lösen in wenig Methylenchlorid und Ausfällen durch Ligroin. Man erhält so

2,5 g (58,4%) 4-[N-(Toluol-4″-sulfonyl)-L-alanyloxy]-4′-di-(n-butyl)-aminodiphenylamin,

farblose Kristalle, Schmp. 118°C; $\alpha_D^{20}$: −53,8°, c = 1% (Aceton).

In analoger Weise erhält man durch Umsetzung der entsprechend substituierten Diphenylamin-Verbindungen (Leuko-Indoaniline) mit den jeweiligen N-geschützten Aminosäuren die folgenden Substrate:

2.1 4-[N-(Toluol-4″-sulfonyl)-L-alanyloxy]-4′-amino-diphenylamin,
amorphes Pulver; $\alpha_D^{20}$: −66,0°, c = 1% (Aceton).
DC: Fertigplatte Kieselgel
(Laufmittel: Toluol-Essigester 1 : 1,
Detektion: UV, $NH_3$ [Gas] und Kaliumhexacyanoferrat-III,
$R_F$-Wert: 0,43).

2.2 4-[N-(Toluol-4″-sulfonyl)-L-alanyloxy]-4′-methylamino-diphenylamin,
amorphes Pulver; $\alpha_D^{20}$: −62,3°, c = 1% (Aceton).
DC: Fertigplatte Kieselgel
(Laufmittel: Heptan-Aceton 2 : 1,
Detektion: UV, $NH_3$ [Gas] und Kaliumhexacyanoferrat-III,
$R_F$-Wert: 0,38).

2.3 4-[N-(Toluol-4″-sulfonyl)-L-alanyloxy]-4′-(2‴-hydroxyethyl)-ethyl-amino-diphenylamin

2.4 4-[N-(Toluol-4″-sulfonyl)-L-alanyloxy]-4′-(N-pyrrolidinyl)-diphenylamin,
farblose Kristalle, Schmp. 139°C; $\alpha_D^{20}$: −58,9°, c = 1% (Aceton).

2.5 4-[N-(Toluol-4″-sulfonyl)-L-alanyloxy]-4′-(N-piperidinyl)-diphenylamin,
amorphes Pulver; $\alpha_D^{20}$: −73,4°, c = 1% (Aceton).
DC: Fertigplatte Kieselgel
(Laufmittel: Heptan-Essigester 3 : 2,
Detektion: UV, $NH_3$ [Gas] und Kaliumhexacyanoferrat-III,
$R_F$-Wert: 0,20).

2.6 4-[N-(Toluol-4″-sulfonyl)-L-alanyloxy]-4′-(N-morpholinyl-diphenylamin,
farblose Kristalle, Schmp. 118°C; $\alpha_D^{20}$: −56,1, c = 1% (Aceton).

2.7 4-[N-(Toluol-4″-sulfonyl)-L-alanyloxy]-2′-brom-4′-dimethylamino-diphenylamin,
farblose Kristalle, Schmp. 107°C; $\alpha_D^{20}$: −54,3°, c = 1% (Aceton).

2.8 4-[N-(Toluol-4″-sulfonyl)-L-alanyloxy]-2′-methyl-4′-dimethylamino-diphenylamin,
schwachgraublaue Kristalle, Schmp. 106°C.

DC: Fertigplatte Kieselgel
(Laufmittel: Chloroform-Methanol 99 : 1,
Detektion: UV, $NH_3$ [Gas] und Kaliumhexacyanoferrat III,
$R_F$-Wert: 0,26).

2.9 4-[N-(Toluol-4''-sulfonyl)-L-alanyl]-2'-trifluormethyl-4'-dimethylamino-diphenylamin, farblose Kristalle, Schmp. 109° C; $\alpha_D^{20}$: −50,1°, c = 1% (Aceton).

2.10 4-[N-Toluol-4''-sulfonyl)-L-alanyloxy]-3'-trifluormethyl-4'-dimethylamino-diphenylamin

2.11 4-[N-(Toluol-4''-sulfonyl)-L-alanyloxy]-2'5'-dimethoxy-4'-dimethylamino-diphenylamin

2.12 4-[N-(Toluol-4''-sulfonyl)-L-alanyloxy]-2'-benzyloxy-4'-dimethylamino-diphenylamin, schwachgraublaues, viskoses Öl.
DC: Fertigplatte Kieselgel
(Laufmittel: Toluol-Essigester 1 : 1,
Detektion: UV, $NH_3$ [Gas] und Kaliumhexacyanoferrat III,
$R_F$-Wert: 0,64).

2.13 4-[N-(Toluol-4''-sulfonyl)-L-alanyloxy]-2'-hydroxy-4'-dimethylamino-diphenylamin, schwachgraue Kristalle, Schmp. 134° C.
DC: Fertigplatte Kieselgel
(Laufmittel: Toluol-Essigester 4 : 1,
Detektion: UV, $NH_3$ [Gas] und Kaliumhexacyanoferrat III,
$R_F$-Wert: 0,49).

2.14 4-[N-(Toluol-4''-sulfonyl)-L-alanyloxy]-2'-nitro-4'-dimethylamino-diphenylamin, schwachrötliches, amorphes Pulver.
DC: Fertigplatte Kieselgel
(Laufmittel: Chloroform-Methanol 98 : 2,
Detektion: UV, $NH_3$ [Gas] und Kaliumhexacyanoferrat III,
$R_F$-Wert: 0,50).

2.15 2-Methyl-4-[N-(toluol-4''-sulfonyl)-L-alanyloxy]-4'-dimethylamino-diphenylamin, farblose Kristalle, Schmp. 134° C; $\alpha_D^{20}$: −5,8°, c = 1% (Aceton).

2.16 3-Brom-4-[N-(toluol-4''-sulfonyl)-L-alanyloxy]-4'-dimethylamino-diphenylamin, farblose Kristalle, Schmp. 124° C; $\alpha_D^{20}$: −46,6°, c = 1% (Aceton).

2.17 3-Methyl-4-[N-(toluol-4''-sulfonyl)-L-alanyloxy]-4'-dimethylamino-diphenylamin, schwachgraublaue Kristalle, Schmp. 97° C.
DC: Fertigplatte Kieselgel
(Laufmittel: Toluol-Essigester 1 : 2,
Detektion: UV, $NH_3$ [Gas] und Kaliumhexacyanoferrat III,
$R_F$-Wert: 0,13).

2.18 3-Methyl-4-[N-(toluol-4''-sulfonyl)-L-alanyloxy]-4'-diethylamino-diphenylamin, farblose Kristalle, Schmp. 79° C; $\alpha_D^{20}$: −67,7°, c = 1% (Aceton).

2.19 2,5-Dimethyl-4-[N-(toluol-4''-sulfonyl)-L-alanyloxy]-4'-dimethylamino-diphenylamin, farblose Kristalle, Schmp. 149° C; $\alpha_D^{20}$: −51,5°, c = 1% (Aceton).

2.20 2-Trifluormethyl-4-[N-(toluol-4''-sulfonyl)-L-alanyloxy]-4'-dimethylamino-diphenylamin, gelbliches, viskoses Öl; $\alpha_D^{20}$: −28,1°, c = 1% (Aceton).
DC: Fertigplatte Kieselgel
(Laufmittel: Toluol-Essigester 2 : 1,
Detektion: UV, $NH_3$ [Gas] und Kaliumhexacyanoferrat III,
$R_F$-Wert: 0,62).

2.21 2-Methoxy-4-[N-(toluol-4''-sulfonyl)-L-alanyloxy]-4'-dimethylamino-diphenylamin, farblose Kristalle, Schmp. 73° C; $\alpha_D^{20}$: −58,0°, c = 1% (Aceton).

2.22 3-Methoxy-4-[N-(toluol-4''-sulfonyl)-L-alanyloxy]-4'-dimethylamino-diphenylamin, farblose Kristalle, Schmp. 130° C; $\alpha_D^{20}$: −40,0°, c = 1% (Aceton).

2.23 2,6-Dimethoxy-4-[N-(toluol-4″-sulfonyl)-L-alanyloxy]-4′-dimethylamino-diphenylamin, farblose Kristalle, Schmp. 127° C; $\alpha_D^{20}$: −50,1°, c=1% (Aceton).

2.24 2-Benzyloxy-4-[N-(toluol-4″-sulfonyl)-L-alanyloxy]-4′-dimethylamino-diphenylamin, schwachgraublaue Kristalle, Schmp. 131° C.
DC: Fertigplatte Kieselgel
(Laufmittel: Toluol-Essigester 2 : 1,
Detektion: UV, $NH_3$ [Gas] und Kaliumhexacyanoferrat III,
$R_F$-Wert: 0,60).

2.25 2-Hydroxy-4-[N-(toluol-4″-sulfonyl)-L-alanyloxy]-4′-dimethylamino-diphenylamin, schwachgraublaue Kristalle, Schmp. 97° C.
DC: Fertigplatte Kieselgel
(Laufmittel: Toluol-Essigester 1 : 1,
Detektion: UV, $NH_3$ [Gas] und Kaliumhexacyanoferrat III,
$R_F$-Wert: 0,46).

2.26 3-Acetylamino-4-[N-(toluol-4″-sulfonyl)-L-alanyloxy]-4′-dimethylamino-diphenylamin, schwachgraue Kristalle, Schmp. 145–148° C.
DC: Fertigplatte Kieselgel
(Laufmittel: Toluol-Essigester 1 : 2,
Detektion: UV, $NH_3$ [Gas] und Kaliumhexacyanoferrat III,
$R_F$-Wert: 0,45).

2.27 3-(Benzoyl-vinyl)-4-[N-(toluol-4″-sulfonyl)-L-alanyloxy]-4′-dimethylamino-diphenylamin, gelbliche Kristalle, Schmp. 146° C; $\alpha_D^{20}$: −77,7°, c=1% (Aceton).

2.28 N-(4′-Diethylamino-phenyl)-8-[N-(toluol-4″-sulfonyl-L-alanyloxy]-chinolyl-5-amin, schwachgraublaue Kristalle, Schmp. 164–166° C.
DC: Fertigplatte Kieselgel
(Laufmittel: Toluol-Essigester 1 : 1,
Detektion: UV, $NH_3$ [Gas] und Kaliumhexacyanoferrat III,
$R_F$-Wert: 0,28).

2.29 N-(4′-Dimethylamino-phenyl)-4-[N-(toluol-4″-sulfonyl)-L-alanyloxy]-naphthyl-1-amin, schwachgraublaues, amorphes Pulver, Schmp. ab 70° C.
DC: Fertigplatte Kieselgel
(Laufmittel: Toluol-Essigester 2 : 1,
Detektion: UV, $NH_3$ [Gas] und Kaliumhexacyanoferrat III,
$R_F$-Wert: 0,45).

2.30 N-(4′-Dimethylamino-phenyl)-3-chlor-4-[N-(toluol-4″-sulfonyl)-L-alanyloxy]-naphthyl-1-amin,
amorphes Pulver.
DC: Fertigplatte Kieselgel
(Laufmittel: Toluol-Essigester 1 : 1,
Detektion: UV, $NH_3$ [Gas] und Kaliumhexacyanoferrat-III,
$R_F$-Wert: 0,67).

2.31 N-(4′-Dimethylamino-phenyl)-3-carboxy-4-[N-(toluol-4″-sulfonyl)-L-alanyloxy]-naphthyl-1-amin,
schwachgraublaue Kristalle, Schmp. 104–106° C.
DC: Fertigplatte Kieselgel
(Laufmittel: Xylol-Methylethylketon 1 : 1 mit 1% Eisessig,
Detektion: UV, $NH_3$ [Gas] und Kaliumhexacyanoferrat-III,
$R_F$-Wert: 0,20).

2.32 N-(4′-Dimethylamino-phenyl)-3-benzyloxycarbonyl-4-[N-(toluol-4″-sulfonyl)-L-alanyloxy]-naphthyl-1-amin,
farblose Kristalle, Schmp. 141° C,
$\alpha_D^{20}$: −55,6°, c=1% (Aceton).

2.33 N-(4′-Dimethylamino-phenyl)-3-carbamoyl-4-[N-(toluol-4″-sulfonyl)-L-alanyloxy]-naphthyl-1-amin

2.34 N-(4′-Dimethylamino-phenyl)-3-(n-butyl-carbamoyl)-4-[N-(toluol-4″-sulfonyl)-L-alanyloxy]-naphthyl-1-amin

2.35 4-[N-(Toluol-4″-sulfonyl)-glycyloxy]-4′-dimethylamino-diphenylamin, farblose Kristalle, Schmp. 126° C.

2.36 4-[N-Toluol-4″-sulfonyl-D-alanyloxy]-4′-dimethylamino-diphenylamin,
schwachgraublaue Kristalle, Schmp. 142° C.
DC: Fertigplatte Kieselgel
(Laufmittel: Chloroform-Methanol 98 : 2,
Detektion: UV, $NH_3$ [Gas] und Kaliumhexacyanoferrat III,
$R_F$-Wert: 0,31).

2.37 4-[N-(Toluol-4″-sulfonyl)-L-seryloxy]-4′-dimethylamino-diphenylamin, farblose Kristalle, Schmp. 141° C; $\alpha_D^{20}$: −45,6°, c = 1% (Aceton).

2.38 4-[N-(Toluol-4″-sulfonyl)-L-valyloxy]-4′-dimethylamino-diphenylamin, farblose Kristalle, Schmp. 131° C; $\alpha_D^{20}$: −46,9°, c = 1% (Aceton).

2.39 2-Methoxy-4-[N-(toluol-4″-sulfonyl)-L-valyloxy]-4′-dimethylamino-diphenylamin,
farbloses, amorphes Pulver; $\alpha_D^{20}$: −44,2°, c = 1% (Aceton).
DC: Fertigplatte Kieselgel
(Laufmittel: Heptan-Essigester 1 : 1,
Detektion: UV, $NH_3$ [Gas] und Kaliumhexacyanoferrat III,
$R_F$-Wert: 0,44).

2.40 2-Methylmercapto-4-[N-(toluol-4″-sulfonyl)-L-valyloxy-4′-dimethylamino-diphenylamin,
schwachgraublaue Kristalle, Schmp. 94° C.
DC: Fertigplatte Kieselgel
(Laufmittel: Toluol-Essigester 2 : 1,
Detektion: UV, $NH_3$ [Gas] und Kaliumhexacyanoferrat-III,
$R_F$-Wert: 0,58).

2.41 2-Methylsulfonyl-4-[N-(toluol-4″-sulfonyl)-L-valyloxy]-4′-dimethylamino-diphenylamin

2.42 4-[N-(Toluol-4″-sulfonyl)-L-leucyloxy]-4′-dimethylamino-diphenylamin,
gelbliches, viskoses Öl; $\alpha_D^{20}$: −30,7°, c = 1% (Aceton).
DC: Fertigplatte Kieselgel
(Laufmittel: Toluol-Essigester 1 : 2,
Detektion: UV, $NH_3$ [Gas] und Kaliumhexacyanoferrat III,
$R_F$-Wert: 0,66).

2.43 4-[N-(Toluol-4″-sulfonyl)-L-methionyloxy]-4′-dimethylamino-diphenylamin,
amorphes Pulver; $\alpha_D^{20}$: −28,1°, c = 1% (Aceton).
DC: Fertigplatte Kieselgel
(Laufmittel: Toluol-Dioxan 9 : 1,
Detektion: UV, $NH_3$ [Gas] und Kaliumhexacyanoferrat-III,
$R_F$-Wert: 0,23).

2.44 4-[N-(Toluol-4″-sulfonyl)-L-phenylalanyloxy]-4′-dimethylamino-diphenylamin, farblose Kristalle, Schmp. 125° C; $\alpha_D^{20}$: −8,5°, c = 1% (Aceton).

2.45 4-[N-(Toluol-4″-sulfonyl)-O-benzyl-L-tyrosyloxy]-4′-dimethylaminodiphenylamin
Das ölig anfallende Rohprodukt wurde ohne weitere Reinigung weiter verarbeitet.
Durch reduktive Abspaltung des Benzylrestes mit Hilfe von Wasserstoff/Platin-Katalysator wird daraus erhalten:

2.46 4-[N-(Toluol-4″-sulfonyl)-L-tyrosyloxy]-4′-dimethylamino-diphenylamin,
schwachgraublaue Kristalle, Schmp. 181 – 183° C.
DC: Fertigplatte Kieselgel
(Laufmittel: Toluol-Essigester 1 : 2,
Detektion: UV, $NH_3$ [Gas] und Kaliumhexacyanoferrat III,
$R_F$-Wert: 0,52).

2.47 4-[N-(Toluol-4″-sulfonyl)-L-alanyl-L-alanyloxy]-4′-dimethylamino-diphenylamin,

farblose Kristalle, Schmp. 155° C; $\alpha_D^{20}$: −46,0°, c = 1% (Aceton).

2.48 4-[N-(Toluol-4″-sulfonyl)-D-alanyl-L-alanyloxy]-4′-dimethylamino-diphenylamin, farblose Kristalle, Schmp. 127° C; $\alpha_D^{20}$: −28,8°, c = 1% (Aceton).

2.49 4-[N-(Toluol-4″-sulfonyl)-L-alanyl-L-alanyl-L-alanyloxy]-4′-dimethylamino-diphenylamin, schwachgraublaue Kristalle, Schmp. 172° C.
DC: Fertigplatte Kieselgel
(Laufmittel: Heptan-Essigester 1 : 8,
Detektion: UV, $NH_3$ [Gas] und Kaliumhexacyanoferrat III,
$R_F$-Wert: 0,25).

2.50 4-(N-Acetyl-L-alanyloxy)-4′-dimethylamino-diphenylamin, farblose Kristalle, Schmp. 142° C; $\alpha_D^{20}$: −7,6°, c = 1% (Aceton).

2.51 4-(N-Benzoyl-D,L-alanyloxy)-4′-dimethylamino-diphenylamin, farblose Kristalle, Schmp. 143° C.

2.52 4-[N-(tert.-Butyloxycarbonyl)-L-alanyloxy]-4′-dimethylamino-diphenylamin, farblose Kristalle, Schmp. 128° C; $\alpha_D^{20}$: −44,8°, c = 1% (Aceton).

2.53 4-[N-(Benzyloxycarbonyl)-L-alanyloxy]-4′-dimethylamino-diphenylamin, farblose Kristalle, Schmp. 116−117° C; $\alpha_D^{20}$: −34,4°, c = 1% (Aceton).

## Beispiel 3

Filterpapier (z. B. Schleicher + Schüll 23 SL) wird nacheinander mit den folgenden Lösungen imprägniert und dann bei 60° C getrocknet.

Lösung 1:

| | |
|---|---|
| Di-natrium-tetraborat-decahydrat | 1,91 g |
| Wasser, dest. | ca. 30 ml |
| Lösung mit 0,1 N-Salzsäure auf einen pH-Wert von 8,0 einstellen, | |
| Wasser, dest., ad | 100,0 ml |

Lösung 2:

| | |
|---|---|
| 4-[N-(Toluol-4″-sulfonyl)-L-alanyloxy]-4′-dimethylamino-diphenylamin | 45,3 mg |
| Aceton, ad | 100,0 ml |

Man erhält ein farbloses Testpapier, welches sich beim Eintauchen in leukozytenhaltige Harne je nach Leukozytenkonzentration hell- bis dunkelblau verfärbt. Es lassen sich nachweisen:

5000 Leukozyten/µl Harn in ca. 2 Minuten
1000 Leukozyten/µl l Harn in ca. 5 Minuten
500 Leukozyten/µl Harn in ca. 8 Minuten
200 Leukozyten/µl Harn in ca. 12 Minuten

Die Empfindlichkeit des Testes liegt bei etwa 200 Leukozyten/µl. Die Beurteilung kann auch remissionsphotometrisch zwischen 570 und 720 nm vorgenommen werden.

Testpapiere mit ähnlichen Eigenschaften (Empfindlichkeiten: 200−2000 Leukozyten/µl) werden erhalten, wenn anstelle von 4-[N-(Toluol-4″-sulfonyl)-L-alanyloxy]-4′-dimethylamino-4′-diphenylamin die folgenden Substrate eingesetzt werden, wobei − wenn nicht anders erwähnt − ebenfalls hell- bis dunkelblaue Verfärbungen der sonst farblosen oder fast farblosen Testpapiere beim Eintauchen in leukozytenhaltige Harne beobachtet werden:

3.1 4-[N-(Toluol-4″-sulfonyl)-L-alanyloxy]-4′-aminodiphenylamin
3.2 4-[N-(Toluol-4″-sulfonyl)-L-alanyloxy]-4′-methylamino-diphenylamin
3.3 4-[N-(Toluol-4″-sulfonyl)-D-alanyloxy]-4′-dimethylamino-diphenylamin
3.4 4-[N-(Toluol-4″-sulfonyl)-L-alanyloxy]-4′-dimethylamino-diphenylamin-hydrochlorid
3.5 4-[N-(Toluol-4″-sulfonyl)-L-alanyloxy]-4′-diethylamino-diphenylamin

3.6 4-[N-(Toluol-4″-sulfonyl)-L-alanyloxy]-4′-(2‴-hydroxyethyl)-ethyl-amino-diphenylamin
3.7 4-[N-(Toluol-4″-sulfonyl)-L-alanyloxy]-4′-(N-pyrrolidinyl)-diphenylamin
3.8 4-[N-(Toluol-4″-sulfonyl)-L-alanyloxy]-4′-(N-piperidinyl)-diphenylamin
3.9 4-[N-(Toluol-4″-sulfonyl)-L-alanyloxy]-4′-(N-morpholinyl)-diphenylamin
3.10 4-[N-(Toluol-4″-sulfonyl)-L-alanyloxy]-4′-dibutylamino-diphenylamin
3.11 3-Acetylamino-4-[N-(toluol-4″-sulfonyl)-L-alanyloxy]-4′-dimethylamino-diphenylamin
3.12 3-(Benzoylvinyl)-4-[N-(toluol-4″-sulfonyl)-L-alanyloxy]-4′-dimethylamino-diphenylamin
3.13 2-Benzyloxy-4-[N-(toluol-4″-sulfonyl)-L-alanyloxy]-4′-dimethylamino-diphenylamin
3.14 3-Brom-4-[N-(toluol-4″-sulfonyl)-L-alanyloxy]-4′-dimethylamino-diphenylamin
3.15 2-Hydroxy-4-[N-(toluol-4″-sulfonyl)-L-alanyloxy]-4′-dimethylamino-diphenylamin
3.16 2-Methoxy-4-[N-(toluol-4″-sulfonyl)-L-alanyloxy]-4′-dimethylamino-diphenylamin
3.17 3-Methoxy-4-[N-(toluol-4″-sulfonyl)-L-alanyloxy]-4′-dimethylamino-diphenylamin
3.18 2,6-Dimethoxy-4-[N-(toluol-4″-sulfonyl)-L-alanyloxy]-4′-dimethylaminodiphenylamin
3.19 2-Methyl-4-[N-(toluol-4″-sulfonyl)-L-alanyloxy]-4′-dimethylamino-diphenylamin
3.20 3-Methyl-4-[N-(toluol-4″-sulfonyl)-L-alanyloxy]-4′-dimethylaminodiphenylamin
3.21 3-Methyl-4-[N-(toluol-4″-sulfonyl)-L-alanyloxy]-4′-diethylamino-diphenylamin
3.22 2,5-Dimethyl-4-[N-(toluol-4″-sulfonyl)-L-alanyloxy]-4′-dimethylaminodiphenylamin
3.23 3,5-Dimethyl-4-[N-(toluol-4″-sulfonyl)-L-alanyloxy]-4′-dimethylaminodiphenylamin
3.24 2,6-Dimethyl-4-[N-(toluol-4″-sulfonyl)-L-alanyloxy]-4′-dimethylaminodiphenylamin
3.25 2-Trifluormethyl-4-[N-(toluol-4″-sulfonyl)-L-alanyloxy]-4′-dimethylaminodiphenylamin
3.26 2′-Benzyloxy-4-[N-toluol-4″-sulfonyl)-L-alanyloxy]-4′-dimethylaminodiphenylamin
3.27 2′-Brom-4-[N-(toluol-4″-sulfonyl)-L-alanyloxy]-4′-dimethylaminodiphenylamin
3.28 2′-Hydroxy-4-[N-(toluol-4″-sulfonyl)-L-alanyloxy]-4′-dimethylaminodiphenylamin
Farbumschlag von rosa nach blau.
3.29 2′,5′-Dimethoxy-4-[N-(toluol-4″-sulfonyl)-L-alanyloxy]-4′-dimethylaminodiphenylamin
3.30 2′-Methyl-4-[N-(toluol-4″-sulfonyl)-L-alanyloxy]-4′-dimethylaminodiphenylamin
3.31 2′-Nitro-4-[N-(toluol-4″-sulfonyl)-L-alanyloxy]-4′-dimethylaminodiphenylamin
Farbumschlag von rot nach blau.
3.32 2′-Trifluormethyl-4-[N-(toluol-4″-sulfonyl)-L-alanyloxy]-4′-dimethylaminodiphenylamin
3.33 3′-Trifluormethyl-4-[N-(toluol-4″-sulfonyl)-L-alanyloxy]-4′-dimethylaminodiphenylamin
3.34 4-[N-(Toluol-4″-sulfonyl)-L-alanyl-L-alanyloxy]-4′-dimethylaminodiphenylamin
3.35 4-[N-(Toluol-4″-sulfonyl)-D-alanyl-L-alanyloxy]-4′-dimethylaminodiphenylamin
3.36 4-[N-(Toluol-4″-sulfonyl)-L-alanyl-L-alanyl-L-alanyloxy]-4′-dimethylamino-diphenylamin
3.37 4-(N-Acetyl-L-alanyloxy)-4′-dimethylamino-diphenylamin
3.38 4-(N-Benzoyl-D,L-alanyloxy)-4′-dimethylamino-diphenylamin
3.39 4-(N-Benzyloxycarbonyl-L-alanyloxy)-4′-dimethylamino-diphenylamin
3.40 4-[N-(tert.-Butyloxycarbonyl)-L-alanyloxy]-4′-dimethylaminodiphenylamin
3.41 4-[N-(Toluol-4″-sulfonyl)-glycyloxy]-4′-dimethylamino-diphenylamin
3.42 4-[N-(Toluol-4″-sulfonyl)-L-leucyloxy]-4′-dimethylamino-diphenylamin
3.43 4-[N-(Toluol-4″-sulfonyl)-L-methionyloxy]-4′-dimethylaminodiphenylamin
3.44 4-[N-(Toluol-4″-sulfonyl)-L-phenylalanyloxy]-4′-dimethylaminodiphenylamin
3.45 4-[N-(Toluol-4″-sulfonyl)-L-seryloxy]-4′-dimethylaminodiphenylamin
3.46 4-[N-(Toluol-4″-sulfonyl)-O-benzyl-L-tyrosyloxy]-4′-dimethylaminodiphenylamin
3.47 4-[N-(Toluol-4″-sulfonyl)-L-tyrosyloxy]-4′-dimethylaminodiphenylamin
3.48 4-[N-(Toluol-4″-sulfonyl)-L-valyloxy]-4′-dimethylaminodiphenylamin
3.49 2-Methylmercapto-4-[N-(toluol-4″-sulfonyl)-L-valyloxy]-4′-dimethylaminodiphenylamin
3.50 2-Methoxy-4-[N-(toluol-4″-sulfonyl)-L-valyloxy]-4′-dimethylaminodiphenylamin
3.51 2-Methylsulfo-4-[N-(toluol-4″-sulfonyl)-L-valyloxy]-4′-dimethylaminodiphenylamin
3.52 N-(4′-Diethylaminophenyl)-8-[N-(toluol-4″-sulfonyl)-L-alanyloxy]-chinolyl-5-amin
Farbumschlag von blaßgrün nach blau.
3.53 N-(4′-Dimethylaminophenyl)-4-[N-(toluol-4″-sulfonyl)-L-alanyloxy]-naphthyl-1-amin
3.54 N-(4′-Dimethylaminophenyl)-3-chlor-4-[N-(toluol-4″-sulfonyl)-L-alanyloxy]-naphthyl-1-amin
3.55 N-(4′-Dimethylaminophenyl)-3-carboxy-4-[N-(toluol-4″-sulfonyl)-L-alanyloxy]-naphthyl-1-amin
3.56 N-(4′-Dimethylaminophenyl)-3-benzyloxycarbonyl-4-[N-(toluol-4″-sulfonyl)-L-alanyloxy]-naphthyl-1-amin
3.57 N-(4′-Dimethylaminophenyl)-3-carbamoyl-4-[N-(toluol-4″-sulfonyl)-L-alanyloxy]-naphthyl-1-amin
3.58 N-(4′-Dimethylaminophenyl)-3-(n-butylcarbamoyl)-4-[N-(toluol-4″-sulfonyl)-L-alanyloxy]-naphthyl-1-amin

Beispiel 4

Filterpapier (z. B. Schleicher und Schüll 23 SL) wird nacheinander mit den folgenden Lösungen imprägniert und dann bei 60° C getrocknet.

Lösung 1:

| | |
|---|---|
| Tris-(hydroxymethyl)-aminomethan | 1,22 g |
| Kaliumhexacyanoferrat (III) | 0,1645 g |
| Wasser, dest. | ca. 50 ml |
| Lösung mit 1 M Salzsäure auf einen pH-Wert von 8,5 einstellen, | |
| Wasser, dest., ad | 100 ml |

Lösung 2:

| | |
|---|---|
| 4-[N-(Toluol-4″-sulfonyl)-L-valyloxy]-4′-dimethylaminodiphenylamin | 48,1 mg |
| Aceton, ad | 100 ml |

Man erhält ein gelbgefärbtes Testpapier, das sich beim Eintauchen in leukozytenhaltige Harne über grün nach blau verfärbt.

Die Empfindlichkeit des Testes liegt bei etwa 300 Leukozyten/μl Harn.

Die Auswertung kann auch remissionsphotometrisch zwischen 570 und 720 nm durchgeführt werden.

Mit den anderen Substraten der Beispiele 1 und 2 werden mit Oxidationsmitteln, wie z. B. dem oben verwendeten Kaliumhexacyanoferrat (III) oder mit Kaliumperoxodisulfat, Kaliummetaperjodat, Natriumperborat und Kaliumchromat Testpapiere erhalten, die gegenüber analogen Testpapieren ohne Oxidationsmitteln z. T. geringfügig verkürzte Reaktionszeiten aufweisen.

Beispiel 5

Filterpapier (z. B. Schleicher und Schüll 23 SL) wird nacheinander mit den folgenden Lösungen imprägniert und dann bei 60° C getrocknet.

Lösung 1:

| | |
|---|---|
| Di-natrium-tetraborat-decahydrat | 7,63 g |
| Wasser, dest. | 80 ml |
| N-(3-Octanoylamidopropyl)-N,N-dimethylammonioacetat | 0,5 g |
| Lösung wird mit 1 M Salzsäure auf einen pH-Wert von 8,0 eingestellt und nach dem Abkühlen auf 25° C | |
| Wasser, dest., ad | 100,0 ml |

Lösung 2:

| | |
|---|---|
| 3-Brom-4-[N-(toluol-4″-sulfonyl)-L-alanyloxy]-4′-dimethylamino-diphenylamin | 53,2 mg |
| Aceton, ad | 100,0 ml |

Man erhält ein farbloses Testpapier, welches sich beim Eintauchen in leukozytenhaltige Harne hell- bis dunkelblau — je nach Leukozytenkonzentration — verfärbt. Gegenüber der Rezeptur des Beispiels 3 ergeben sich etwas verkürzte Reaktionszeiten und geringfügig brillantere Farben.

Auch mit den anderen Substraten der Beispiele 1 und 2 werden mit Netzmitteln, wie beispielsweise dem oben verwendeten (amphoter) aber auch z. B. mit Nonylphenolpolyglykolether (nichtionogen), oder Benzyltrimethylammoniumchlorid (kationenaktiv) oder Natriumsulfonato-dodecylbenzol (anionenaktiv), Testpapiere erhalten, die gegenüber analogen Testpapieren ohne Netzmittel z. T. geringfügig verkürzte Reaktionszeiten und etwas brillantere Farben aufweisen.

Beispiel 6

Filterpapier (z. B. Schleicher und Schüll 2316) wird nacheinander mit den folgenden Lösungen imprägniert und dann bei 60° C getrocknet:

Lösung 1:

| | |
|---|---|
| Dinatriumtetraboratdecahydrat | 3,81 g |
| Wasser, dest. | ca. 80 ml |
| Lösung wird mit 1 M Salzsäure auf einen pH-Wert von 8,0 eingestellt. | |
| Wasser, dest., ad | 100,0 ml |

Lösung 2:

| | |
|---|---|
| 4-[N-(Toluol-4''-sulfonyl)-L-alanyloxy]-4'-(N-pyrrolidinyl)-diphenylamin | 47,9 mg |
| tert.-Butylhydrochinon | 8,8 mg |
| 3-(tert.-Butyl)-4-hydroxyanisol | 9,0 mg |
| Aceton, ad | 100,0 ml |

Man erhält ein weißes Testpapier, welches sich beim Eintauchen in leukozytenhaltige Harne hell- bis dunkelblau — je nach der Leukozytenkonzentration — verfärbt.

Auch mit den anderen Substraten der Beispiele 1 und 2 sowie mit anderen Antioxidantien, wie Methyl- und Dimethylhydrochinon, 2,6-Di-(tert.-butyl)-4-methylphenol, 2,4,6-Tri-(tert.-butyl)-phenol, 4-Methoxyphenol oder (2-Methoxy-2-methyl-1-propyl)-hydrochinon, werden weiße Testpapiere erhalten, die sich im Vergleich zu analogen Testpapieren ohne Antioxidantien auch bei längerem Lagern nicht blau verfärben.

Die geringfügige Verzögerung der Farbreaktion beim Eintauchen in leukozytenhaltige Harne kann durch Mitimprägnieren der in Beispiel 4 genannten Oxidationsmittel beseitigt werden.

Beispiel 7

Filterpapier (z. B. Schleicher und Schüll 23 SL) wird nacheinander mit den folgenden Lösungen imprägniert und dann bei 60° C getrocknet:

Lösung 1:

| | |
|---|---|
| Dinatriumtetraboratdecahydrat | 7,63 g |
| Wasser, dest. | ca. 70 ml |
| Phosphorsäuretrimorpholid | 10,0 g |
| Die Lösung wird mit 1 M Salzsäure auf einen pH-Wert von 8,0 eingestellt und nach dem Abkühlen auf 25° C | |
| Wasser, dest., ad | 100,0 ml |

Lösung 2:

| | |
|---|---|
| 3-Methyl-4-[N-(toluol-4''-sulfonyl)-L-alanyloxy]-4'-diethylaminodiphenylamin | 74,3 mg |
| Aceton, ad | 100,0 ml |

Man erhält ein schwachgelblichgefärbtes Testpapier, welches sich beim Eintauchen in leukozytenhaltige Harne je nach der Leukozytenkonzentration hell- bis dunkelblau verfärbt.

Auch mit den anderen Substraten der Beispiele 1 und 2 sowie mit anderen Phosphorsäurederivaten, wie

Phosphorsäuretrismethylamid,
Phorphorsäuremonoethylesterdimorpholid,
Phosphorsäuremonophenylesterdimorpholid,
Ethylphosphonsäuredimorpholid,
Phenylphosphonsäurebisdiäthylamid,
Phenylphosphonsäuredimorpholid,

wurden weiße bis schwachgelbliche Papiere erhalten, die sich im Vergleich zu analogen Testpapieren ohne Phosphorsäure-Derivate durch eine deutlich erhöhte Hydrolysebeständigkeit der imprägnierten Substrate auszeichneten.

Ferner gelang es, mit Hilfe der in diesem Beispiel erwähnten Phosphorsäure-Derivate Oxidationsmittel des Beispiels 4 neben Reduktionsmitteln (Antioxidantien) des Beispiels 6 in eine stabile Rezeptur einzubauen.

## Beispiel 8

Filterpapier (z. B. Schleicher und Schüll 23 SL) wird nacheinander mit den folgenden Lösungen imprägniert und dann bei 60° C bzw. Raumtemperatur getrocknet.

Lösung 1:

| | |
|---|---|
| Dinatriumtetraboratdecahydrat | 1,91 g |
| Wasser, dest. | ca. 60 ml |
| Lösung wird mit 1 M Salzsäure auf einen pH-Wert von 8,0 eingestellt. | |
| Wasser, dest., ad | 100,0 ml |

Lösung 2:

Substratlösungen $1 \times 10^{-3}$ M in Aceton z. A.

Die erfindungsgemäßen Aktivatoren der allgemeinen Formel (V) werden der Lösung 2 zugesetzt, so daß eine Endkonzentration von 2% (w/v) resultiert, von Aktivatoren der allgemeinen Formel (VI) werden 0,5 mMol in der Lösung 1 gelöst.

In der Tabelle 1 sind die Versuchsergebnisse mit folgenden Esterase- oder Protease-Substraten zusammengestellt:

A: 4-[N-(Toluol-4″-sulfonyl)-L-alanyloxy]-4′-dimethylaminodiphenylamin
B: 3-Brom-4-[N-(toluol-4″-sulfonyl)-L-alanyloxy]-4′-dimethylaminodiphenylamin
C: 4-[N-(Toluol-4″-sulfonyl)-L-alanyloxy]-3′-trifluormethyl-4′-dimethylaminodiphenylamin
D: 4-[N-(Toluol-4″-sulfonyl)-L-valyloxy]-4′-dimethylaminodiphenylamin

In der Tabelle 1 sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswerte dienen die Reaktionszeiten der jeweiligen Rezepturen ohne Aktivatorzusätze.

Der Farbumschlag bei den vier hier untersuchten Verbindungen erfolgt von farblos nach tiefblau.

Tabelle 1

| Aktivatoren | Reaktionszeiten für Substrate | | | |
|---|---|---|---|---|
| | A | B | C | D |
| Vergleichsrezeptur ohne Aktivatoren | 120 s | 230 s | 215 s | 180 s |
| 1. 1-Hexanol | 70 s | 200 s | 180 s | 100 s |
| 2. 1-Heptanol | 75 s | 180 s | 180 s | 110 s |
| 3. 1-Octanol | 75 s | 210 s | 190 s | 90 s |
| 4. 1-Nonanol | 65 s | 200 s | 170 s | 90 s |
| 5. 1-Decanol | 35 s | 175 s | 150 s | 80 s |
| 6. 1-Dodecanol | 50 s | 190 s | 160 s | 120 s |
| 7. 1-Tetradecanol | 32 s | 140 s | 140 s | 70 s |
| 8. 1-Pentadecanol | 40 s | 160 s | 145 s | 95 s |
| 9. 1-Hexadecanol | 45 s | 160 s | 170 s | 90 s |
| 10. 1-Heptadecanol | 55 s | 150 s | 180 s | 105 s |
| 11. 1-Octadecanol | 50 s | 180 s | 200 s | 110 s |
| 12. 1-Nonadecanol | 60 s | 210 s | 200 s | 110 s |
| 13. 1-Eicosanol | 55 s | 190 s | 210 s | 120 s |
| 14. 1-Docosanol | 75 s | 180 s | 200 s | 120 s |
| 15. Cyclohexanol | 90 s | 190 s | 150 s | 120 s |
| 16. 1-Cydohexen-3-ol | 110 s | 210 s | 160 s | 150 s |
| 17. Cycloheptanol | 90 s | 195 s | 180 s | 120 s |
| 18. Cyclooctanol | 85 s | 205 s | 180 s | 110 s |
| 19. Cyclononanol | 80 s | 210 s | 150 s | 110 s |
| 20. Cyclodecanol | 105 s | 200 s | 180 s | 100 s |
| 21. Cyclododecanol | 100 s | 190 s | 180 s | 100 s |
| 22. Cycloheptadecanol | 110 s | 205 s | 190 s | 110 s |
| 23. 9-Cycloheptadecen-1-ol | 55 s | 180 s | 170 s | 110 s |
| 24. Citronellol | 35 s | 160 s | 150 s | 90 s |
| 25. Geraniol | 45 s | 190 s | 150 s | 95 s |
| 26. Nerol | 60 s | 190 s | 160 s | 130 s |
| 27. Linalool | 75 s | 200 s | 180 s | 110 s |
| 28. Farnesol | 55 s | 180 s | 160 s | 110 s |

Fortsetzung

| Aktivatoren | Reaktionszeiten für Substrate | | | |
|---|---|---|---|---|
| | A | B | C | D |
| 29. Nerolidol | 60 s | 190 s | 190 s | 100 s |
| 30. (Z)-9-Octadecen-1-ol | 40 s | 160 s | 140 s | 60 s |
| 31. Phytol | 45 s | 155 s | 150 s | 80 s |
| 32. 1,5-Pentadiol | 70 s | 230 s | 200 s | 150 s |
| 33. 1,6-Hexandiol | 70 s | 220 s | 210 s | 150 s |
| 34. 1,7-Heptandiol | 105 s | 225 s | 200 s | 130 s |
| 35. 1,8-Octandiol | 90 s | 230 s | 215 s | 100 s |
| 36. 1,9-Nonandiol | 95 s | 240 s | 220 s | 110 s |
| 37. 1,10-Decandiol | 110 s | 240 s | 220 s | 160 s |
| 38. 1,12-Dodecandiol | 110 s | 230 s | 230 s | 180 s |
| 39. 2-Butyloxyethanol | 80 s | 205 s | 200 s | 100 s |
| 40. 2-(2-Butyloxyethyloxy)ethanol | 95 s | 200 s | 190 s | 120 s |
| 41. Trikaliumhexacyanoferrat (III) | 110 s | 285 s | 200 s | 180 s |
| 42. Tetrakaliumhexacyanoferrat (II) | 130 s | 200 s | 180 s | 160 s |
| 43. Dikaliumtetracyanonickelat (II) | 150 s | 210 s | 210 s | 160 s |
| 44. Trinatriumoctacyanomolybdat (V) | 120 s | 230 s | 200 s | 170 s |
| 45. Dinatriumpentacyanonitrosylferrat (II) | 90 s | 175 s | 170 s | 160 s |
| 46. Trikaliumpentacyanonitrosylmanganat (I) | 80 cs | 160 s | 215 s | 150 s |
| 47. Trikaliumpentacyanonitrosylchromat (I) | 120 s | 200 s | 200 s | 150 s |
| 48. Trikaliumpentacyanonitrosylcobaltat (I) | 100 s | 240 s | 195 s | 190 s |
| 49. Pentakaliumpentacyanonitrosylvanadat (I) | 140 s | 260 s | 200 s | 190 s |

Ähnliche Versuchsergebnisse werden mit den anderen Substraten der Beispiele 1 und 2 und/oder leukozytenhaltigen Harnen anstelle der Standardlösung von 5000 Leukozyten/µl isotonischer Kochsalzlösung erzielt.

Beispiel 9

Filterpapier (z. B. Schleicher und Schüll 23 SL) wird nacheinander mit den folgenden Lösungen imprägniert und dann bei 60° C bzw. Raumtemperatur getrocknet.

Lösung 1:

| | |
|---|---|
| Dinatriumtetraboratdecahydrat | 1,91 g |
| Wasser, dest. | ca. 60 ml |
| Lösung wird mit 1 M Salzsäure auf pH 8,5 eingestellt | |
| Wasser, dest., ad | 100,0 ml |

Lösung 2:

| | |
|---|---|
| 2-Methoxy-4-[N-(toluol-4″-sulfonyl)-L-valyloxy]-4′-dimethylaminodiphenylamin | 51,1 mg |
| Aceton, ad | 100,0 ml |

Die erfindungsgemäßen Aktivatoren werden einzeln oder als Gemisch je nach der Löslichkeit der Lösung 1 und/oder Lösung 2 zugesetzt, so daß bei den einzelnen Aktivatoren der allgemeinen Formeln (V) und (VI) jeweils Endkonzentrationen von 2% (w/v) bzw. $5 \times 10^{-3}$ M resultieren.

In Tabelle 2 sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswert dient die Reaktionszeit der Rezeptur ohne Aktivatorzusätze.

Die Testpapiere verfärben sich beim Eintauchen in leukozytenhaltige Lösungen von weiß nach tiefblau.

Tabelle 2

| Aktivatoren | Reaktionszeiten |
|---|---|
| Vergleichsrezeptur ohne Aktivatoren | 210 s |
| 1. 1-Decanol | 95 s |
| 2. 1-Tetradecanol | 90 s |
| 3. Dinatriumpentacyanonitrosulferrat (II) | 130 s |
| 4. Tetrakaliumhexacyanoferrat (II) | 150 s |
| Aktivatoren 1 und 3 | 75 s |
| Aktivatoren 1 und 4 | 80 s |
| Aktivatoren 2 und 3 | 60 s |
| Aktivatoren 2 und 4 | 65 s |

Ähnliche Versuchsergebnisse werden mit den anderen Substraten der Beispiele 1 und 2 und/oder leukozytenhaltigen Harnen anstelle der Standardlösung von 5000 Leukozyten/µl isotonischer Kochsalzlösung erhalten.

## Beispiel 10

Eine Tablette, enthaltend

| | |
|---|---|
| 4-(N-Benzoyl-D,L-alanyloxy)-4′-dimethylaminodiphenylamin | 3,0 mg |
| Kaliumdihydrogenphosphat | 1,5 mg |
| Di-natriumhydrogenphosphat-dihydrat | 30,0 mg |
| Mannit, ad | 90,0 mg |

wird zu 2 ml eines leukozytenhaltigen Harnes in einem Reagenzglas gegeben. Der Harn verfärbt sich allmählich – je nach Leukozytenkonzentration – hellgrün bis tiefblau.

Nach 10 Minuten Stehenlassen bei Raumtemperatur wird die Leukozytenkonzentration visuell mit Hilfe von Vergleichsfarben bestimmt oder photometrisch, z. B. in 1-cm-Mikro-Küvetten bei 660 nm.

Die Empfindlichkeit des Testes liegt bei etwa 390 Leukozyten/µl Harn.

Mit den anderen Substraten der Beispiele 1 und 2 lassen sich ähnliche Empfindlichkeiten (300–1000 Leukozyten/µl) erreichen, wobei sich bei schwerlöslichen Substraten die Zugabe organischer Lösungsmittel, wie z. B. Methanol oder Dimethylformamid, empfiehlt.

Mit Oxidationsmitteln, Netzmitteln, Antioxidantien, Stabilisatoren und/oder Aktivatoren werden auch bei Reagenztabletten vergleichbare Effekte erzielt, wie sie in den Beispielen 4–9 für Teststreifen beschrieben sind.

Beispiel 11

Filterpapier (z. B. Schleicher + Schüll 23 SL) wird nacheinander mit den folgenden Lösungen imprägniert und dann bei 60° C getrocknet.

Lösung 1:

| | |
|---|---|
| Di-natrium-tetraborat-decahydrat | 1,91 g |
| Wasser, dest. | ca. 30 ml |
| Lösung wird mit 0,1 N-Salzsäure auf einen pH-Wert von 8,0 eingestellt, | |
| Wasser, dest., ad | 100,0 ml |

Lösung 2:

| | |
|---|---|
| 4-[N-(Toluol-4″-sulfonyl)-L-phenylalanyloxy]-4′-dimethylaminodiphenylamin | 51,5 mg |
| Aceton, ad | 100,0 ml |

Man erhält ein farbloses Testpapier, welches sich beim Eintauchen in wäßrige Lösungen, die das proteolytische Enzym Chymotrypsin enthalten, blau verfärbt. Es lassen sich so noch Konzentrationen von 0,005 U Chymotrypsin pro ml in ca. 2–4 Minuten nachweisen.

(Die angegebene Enzymaktivität wurde mit N-Acetyl-L-tyrosin-ethylester als Substrat bei 25° C, pH 7,0 und $\lambda = 237$ nm bestimmt.)

Auch mit den anderen Substraten der Beispiele 1 und 2 lassen sich – je nach Aminosäure- bzw. Peptidrest – Chymotrypsin oder andere proteolytische Enzyme, wie z. B. Elastase oder Trypsin, in rein wäßrigen Lösungen oder auch z. B. in Körperflüssigkeiten, wie z. B. Vollblut, Serum, Liquor, Pankreassekret der wäßrigen Stuhlextrakten, nachweisen.

**Patentansprüche**

1. Aminosäure- und Peptidester von Leuko-Indoanilinen der allgemeinen Formel I

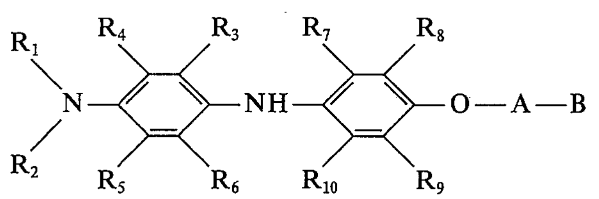

(I)

in der

$R_1$, $R_2$, die gleich oder verschieden sein können, jeweils Wasserstoff, eine $C_1-C_6$-Alkyl- oder eine Hydroxy-$C_1-C_6$-Alkyl-Gruppe oder gemeinsam eine Kohlenwasserstoff-Kette mit 1–6 Kohlenstoffatomen, die Sauerstoff- bzw. Stickstoffatome als Kettenglieder enthalten kann,

$R_3$, $R_4$, $R_5$, $R_6$, die gleich oder verschieden sein können, jeweils Wasserstoff oder Halogen, eine gegebenenfalls vollständig durch Halogen substituierte $C_1-C_6$-Alkyl-, $C_1-C_6$-Alkoxy-, einen durch $C_1-C_5$-Alkoxy substituierten Phenyl- oder Naphthyl-Rest, eine Hydroxy- oder eine Nitro-Gruppe,

$R_7$, $R_8$, $R_9$, $R_{10}$, die gleich oder verschieden sein können, jeweils Wasserstoff oder Halogen, eine gegebenenfalls vollständig durch Halogen substituierte $C_1-C_6$-Alkyl-, eine $C_1-C_6$-Alkoxy-, einen durch $C_1-C_5$-Alkoxy substituierten Phenyl- oder Naphthylrest, eine $C_1-C_5$-Alkanoyl-amino-, eine Benzoyl- oder Naphthoyl-$C_2-C_5$-alkenyl-, eine Hydroxy-, eine $C_1-C_6$-Alkylmercapto-, eine $C_1-C_6$-Alkylsulfonyl-, eine Carboxy-, eine Carbonyl-Gruppe, die gegebenenfalls durch eine $C_1-C_6$-Alkoxy-, eine durch $C_1-C_5$-Alkoxy substituierte Phenyl- oder Naphthyl-, eine Amino- oder $C_1-C_6$-Alkylamino-Gruppe substituiert ist, oder jeweils zwei benachbarte Substituenten eine gesättigte oder ungesättigte Kohlenwasserstoffkette mit 1–6 Kohlenstoffatomen, wobei ein Kettenglied durch ein Stickstoffatom ersetzt sein kann,

A einen Aminosäure- oder einen Peptidrest,

B eine in der Peptidchemie übliche oder davon abgeleitete Stickstoffschutzgruppe bedeutet.

2. Verfahren zur Herstellung von Aminosäure- und Peptidestern von Leuko-Indoanilinen gemäß Anspruch 1, dadurch gekennzeichnet, daß man in an sich bekannter Weise Verbindungen der allgemeinen Formel II

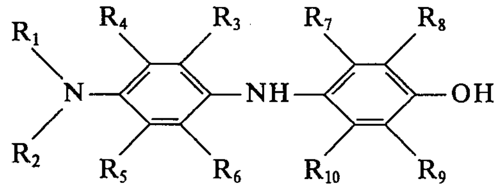

(II)

in der

$R_1$, $R_2$, $R_3$, $R_4$, $R_5$, $R_6$, $R_7$, $R_8$, $R_9$ und $R_{10}$ die in Anspruch 1 angegebene Bedeutung haben,

mit Aminosäuren und Peptiden der allgemeinen Formel III

$$HO-A-B \qquad (III)$$

in der

A und B die in Anspruch 1 angegebene Bedeutung haben,

beziehungsweise mit reaktiven Derivaten davon, umsetzt.

3. Verwendung von Verbindungen gemäß Anspruch 1 zur Herstellung von Mitteln zum Nachweis proteolytischer Enzyme.

4. Mittel zum Nachweis proteolytischer Enzyme, enthaltend ein oder mehrere Chromogene, eine geeignete Puffersubstanz sowie gegebenenfalls Hilfsstoffe und Aktivatoren, dadurch gekennzeichnet, daß als Chromogene Aminosäureester und/oder Peptidester von Leuko-Indoanilinen gemäß Anspruch 1 eingesetzt werden.

5. Mittel gemäß Anspruch 4, dadurch gekennzeichnet, daß als Hilfsstoffe Netzmittel, Oxidationsmittel, Antioxidantien, Stabilisatoren, Filmbildner, galenische Zusatzstoffe und/oder Gerüstbildner verwendet werden.

6. Mittel gemäß Anspruch 5, dadurch gekennzeichnet, daß als Stabilisatoren Phosphor- bzw. Phosphonsäureamide der allgemeinen Formel IV

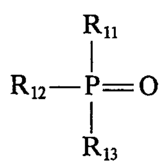

(IV)

in der

$R_{11}$ eine Dialkylamino-, eine Alkoxy-, eine Aryloxy-, eine Alkyl- oder eine Arylgruppe oder einen N-Morpholinrest und
$R_{12}$ und $R_{13}$ eine Dialkylaminogruppe oder einen N-Morpholinrest bedeuten,

verwendet werden.

7. Mittel gemäß einem der Ansprüche 4–6, dadurch gekennzeichnet, daß als Aktivatoren Alkohole der allgemeinen Formel V

$$R_{14}-K-OH \qquad (V)$$

in der

$R_{14}$ Wasserstoff, eine Hydroxy- oder eine niedere Alkoxygruppe und
K einen Kohlenwasserstoffrest bedeuten,

und/oder Metallkomplexe der allgemeinen Formel VI

$$D_m[M(CN)_n(NO)_p] \qquad (VI)$$

in der

D ein Alkalimetallion,
M ein Schwermetallion,
m eine ganze Zahl von 2 bis 5,
n eine ganze Zahl von 4 bis 8 und
p 0 oder 1 bedeuten,

verwendet werden.

8. Mittel gemäß einem der Ansprüche 4–7, dadurch gekennzeichnet, daß ein saugfähiger Träger mit den Inhaltsstoffen imprägniert wird.

9. Mittel gemäß einem der Ansprüche 4–7, dadurch gekennzeichnet, daß die Inhaltsstoffe in einer Reagenztablette enthalten sind.

## Claims

1. Amino acid and peptide esters of leuko-indoanilines of the general formula I

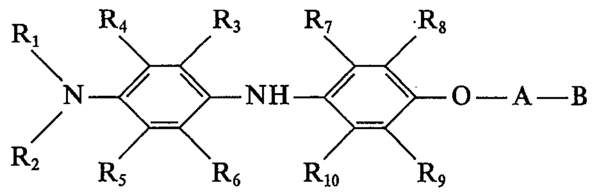

(I)

in which

$R_1$, $R_2$, which can be the same or different, each signify hydrogen, a $C_1-C_6$-alkyl or a hydroxy-$C_1-C_6$-alkyl group or together a hydrocarbon chain with 1–6 carbon atoms, which can contain oxygen or nitrogen atoms as chain members,

$R_3$, $R_4$, $R_5$, $R_6$, which can be the same or different, each signify hydrogen or halogen, a $C_1-C_6$-alkyl possibly completely substituted by halogen, $C_1-C_6$-alkoxy, a phenyl or naphthyl radical substituted by $C_1-C_5$-alkoxy, a hydroxyl or a nitro group,

$R_7$, $R_8$, $R_9$, $R_{10}$, which can be the same or different, each signify hydrogen or halogen, a $C_1-C_6$-alkyl possibly completely substituted by halogen, a $C_1-C_6$-alkoxy, a phenyl or naphthyl radical substituted by $C_1-C_5$-alkoxy, a $C_1-C_5$-alkanoylamino, a benzol or naphthoyl-$C_2-C_5$-alkenyl, a hydroxyl, a $C_1-C_6$-alkylmercapto, a $C_1-C_6$-alkylsulphonyl, a carboxyl, a carbonyl group, which is possibly substituted by $C_1-C_6$-alkoxy, a phenyl or naphthyl substituted by $C_1-C_5$-alkoxy, an amino or $C_1-C_6$-alkylamino group, or two neighbouring substituents signify a saturated or unsaturated hydrocarbon chain with 1–6 carbon atoms, whereby a chain member can be replaced by a nitrogen atom,

A signifies an amino acid or a peptide residue,

B signifies a nitrogen protective group conventional in peptide chemistry or derived therefrom.

2. Process for the preparation of amino acid and peptide esters of leuko-indoanilines according to claim 1, characterised in that in per se known manner, one reacts compounds of the general formula II

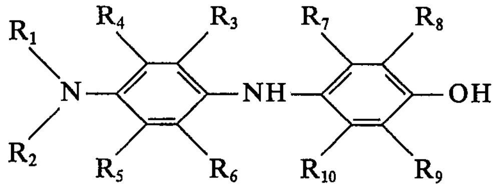

(II)

in which

$R_1$, $R_2$, $R_3$, $R_4$, $R_5$, $R_6$, $R_7$, $R_8$, $R_9$ and $R_{10}$ have the meaning given in claim 1,

with amino acids and peptides of the general formula III

$$HO-A-B \quad (III)$$

in which

A and B have the meaning given in claim 1,

or with reactive derivatives thereof.

3. Use of compounds according to claim 1 for the production of agents for the detection of proteolytic enzymes.

4. Agent for the detection of proteolytic enzymes, containing one or more chromogens, a suitable buffer substance, as well as possibly adjuvant materials and activators, characterised in that, as

chromogens, there are used amino acid esters and/or peptide esters of leuko-indoanilines according to claim 1.

5. Agent according to claim 4, characterised in that, as adjuvant material, there are used wetting agents, oxidation agents, anti-oxidants, stabilisers, film formers, galenical additive materials and/or structure formers.

6. Agent according to claim 5, characterised in that as stabilisers there are used phosphoric or phosphonic acid amides of the general formula IV

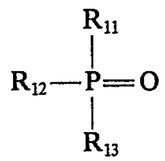 (IV)

in which

$R_{11}$ signifies a dialkylamino, an alkoxy, an aryloxy, an alkyl or an aryl group or an N-morpholine radical and

$R_{12}$ and $R_{13}$ signify a dialkylamino group or an N-morpholine residue.

7. Agent according to one of claims 4–6, characterised in that, as activators, there are used alcohols of the general formula V

$$R_{14}-K-OH \qquad (V)$$

in which

$R_{14}$ signifies hydrogen, a hydroxyl or a lower alkoxy group and

K a hydrocarbon residue,

and/or metal complexes of the general formula VI

$$D_m[M(CN)_n(NO)_p] \qquad (VI)$$

in which

D signifies an alkali metal ion,

M a heavy metal ion,

m a whole number of 2 to 5,

n a whole number of 4 to 8 and

p 0 or 1.

8. Agent according to one of claims 4–7, characterised in that an absorbent carrier is impregnated with the component materials.

9. Agent according to one of claims 4–7, characterised in that the component materials are contained in a reagent tablet.

**Revendications**

1. Esters d'acides aminés et de peptides de leuco-indo-anilines de formule générale I

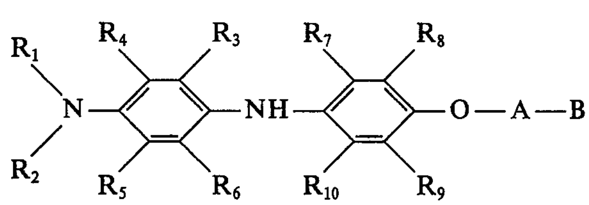 (I)

dans laquelle

$R_1$, $R_2$, pouvant être identiques ou différents sont chacun de l'hydrogène, un groupe alkyle $C_1-C_6$ ou un groupe hydroxy-alkyle $C_1-C_6$ ou forment ensemble un chaîne hydrocarbonée ayant de 1 à 6 atomes de carbone pouvant renfermer dans la chaîne des atomes d'oxygène ou d'azote,

$R_3$, $R_4$, $R_5$, $R_6$, pouvant être identiques ou différents sont chacun de l'hydrogène ou de l'halogène, un

reste alkyle $C_1-C_6$ éventuellement complètement substitué par de l'halogène, un reste alcoxy $C_1-C_6$, un reste phényle ou naphtyle substitué par alcoxy $C_1-C_5$, un groupe hydroxyle ou nitro,

$R_7$, $R_8$, $R_9$, $R_{10}$, pouvant ête identiques ou différents, sont chacun de l'hydrogène ou de l'halogène, un reste alkyle $C_1-C_6$ éventuellement complètement substitué par de l'halogène, un reste alcoxy $C_1-C_6$, un reste phényle ou naphtyle substitué par alcoxy $C_1-C_5$, un groupe alcanoyle $C_1-C_5$-amino, benzoyl-alcényle $C_2-C_5$, naphtoyl-alcényle $C_2-C_5$, hydroxyle, alkyl-mercapto $C_1-C_6$, alkylsulfonyle $C_1-C_6$, carboxyle, carbonyle éventuellement substitué par alcoxy $C_1-C_6$, un groupe phényle ou naphtyle substitué par de l'alcoxy $C_1-C_5$, ou encore par un groupe amino ou alkyle $C_1-C_6$-amino, ou bien chaque fois deux substituants forment une chaîne hydrocarbure saturée ou insaturée ayant de 1 à 6 atomes de carbone, un maillon de la chaîne pouvant être remplacé par un atome d'azote,

A est un acide aminé ou un reste peptide,

B est un groupe de protection de l'azote habituellement utilisé dans la chimie des peptides, ou un dérivé d'un tel groupe.

2. Procédé de préparation d'esters d'acides aminés et de peptides de leuco-indo-anilines selon la revendication 1, caractérisé en ce que de façon en soi connue on fait réagir des composés de formule générale II

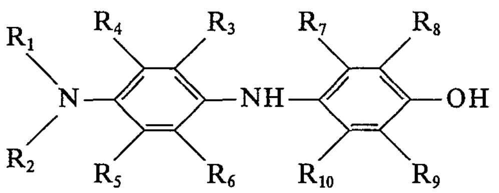

(II)

dans laquelle

$R_1$, $R_2$, $R_3$, $R_4$, $R_5$, $R_6$, $R_7$, $R_8$, $R_9$ et $R_{10}$ ont la signification donnée dans la revendication 1,

avec des acides aminés et des peptides de formule générale III

$$HO-A-B \quad \text{(III)}$$

dans laquelle

A et B ont la signification donnée dans la revendication 1,

ou avec des dérivés réactifs de ces composés.

3. Utilisation des composés selon la revendication 1 pour la fabrication d'agents pour la mise en évidence d'enzymes protéolytiques.

4. Agent pour la mise en évidence d'enzymes protéolytiques contenant un ou plusieurs chromogènes, une substance tampon appropriée ainsi qu'éventuellement des agents auxiliaires et activateurs, caractérisé en ce qu'en tant que chromogènes on utilise des esters d'acides aminés et/ou des esters de peptides de leuco-indo-anilines selon la revendication 1.

5. Agent selon la revendication 4, caractérisé en ce qu'on utilise comme agents auxiliaires des agents mouillants, des agents oxydants, des agents anti-oxydants, des stabilisants, des formateurs de films, des additifs galéniques et/ou des formateurs de structures.

6. Agent selon la revendication 5, caractérisé en ce qu'on utilise comme stabilisants des amides d'acide phosphorique ou phosphonique de formule générale IV

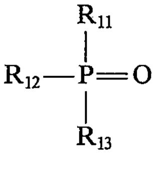

(IV)

dans laquelle

$R_{11}$ est un groupe dialkylamino, alcoxy, aryloxy, alkyle ou aryle ou un reste N-morpholino,

$R_{12}$ et $R_{13}$ sont un groupe dialkylamino ou un reste N-morpholino.

7. Agent selon l'une des revendications 4 à 6, caractérisé en ce qu'on utilise comme activateurs des alcools de formule générale V

$$R_{14}-K-OH \quad \text{(V)}$$

dans laquelle

$R_{14}$ est de l'hydrogène, un groupe hydroxyle ou un groupe alcoxy inférieur et
K est un reste hydrocarbure,

et/ou des complexes métalliques de formule générale VI

$$D_m[M(CN)_n(NO)_p] \quad (VI)$$

dans laquelle

D est un ion de métal alcalin,
M est un ion de métal lourd,
m est un nombre entier de 2 à 5,
n est un nombre entier de 4 à 8, et
p est 0 ou 1.

8. Agent selon l'une des revendications 4 à 7, caractérisé en ce qu'un support absorbant est imprégné des substances constituantes.

9. Agent selon l'une des revendications 4 à 7, caractérisé en ce que les substances constituantes sont contenues dans un comprimé de réactifs.